# EUROPÄISCHE PATENTSCHRIFT

(11) **EP 1 257 172 B1**
(45) Veröffentlichungstag und Bekanntmachung des Hinweises auf die Patenterteilung: **13.10.2004**
(21) Anmeldenummer: 01919332.5
(22) Anmeldetag: 23.02.2001
(51) Int. Cl.: A01N 37/32, A01N 41/10, A01N 37/38, A01N 37/36, A01N 41/02, A01N 43/36, C07D 207/50

(54) **FUNGIZIDE MITTEL ENTHALTEND ALS WIRKSTOFFE PYRROLIDONE UND DEREN VERWENDUNG BEI DER BEHANDLUNG VON PFLANZEN**
FUNGICIDAL AGENTS CONTAINING PYRROLIDONES AS THEIR ACTIVE AGENTS AND USE THEREOF FOR TREATING PLANTS
PRODUIT FONGICIDE CONTENANT DES PYRROLIDONES COMME PRINCIPES ACTIFS ET LEUR UTILISATION LORS DU TRAITEMENT DE PLANTES

(30) Priorität: 26.02.2000 DE 10009115
(43) Veröffentlichungstag der Anmeldung: 20.11.2002
(73) Patentinhaber: BASF AKTIENGESELLSCHAFT, 67056 Ludwigshafen (DE)
(72) Erfinder: RHEINHEIMER, Joachim, 67063 Ludwigshafen (DE); EICKEN, Karl, 67157 Wachenheim (DE); ROSE, Ingo, 68159 Mannheim (DE); GROTE, Thomas, 67157 Wachenheim (DE); AMMERMANN, Eberhard, 64646 Heppenheim (DE); SPEAKMAN, John-Bryan, 67273 Bobenheim (DE); STRATHMANN, Siegfried, 67117 Limburgerhof (DE); LORENZ, Gisela, 67434 Neustadt (DE)
(86) Internationale Anmeldenummer: PCT/EP2001/002059
(87) Internationale Veröffentlichungsnummer: WO 2001/062087

(56) Entgegenhaltungen:
- WO-A-00/30445
- DATABASE CHEMABS [Online] CHEMICAL ABSTRACTS SERVICE, COLUMBUS, OHIO, US; retrieved from STN-INTERNATIONAL, accession no. 84:70326 CA XP002176192 & JP 50 117929 A (KUREHA CHEMICAL INDUSTRY) 16. September 1975 (1975-09-16)
- AUGUSTIN M ET AL: "UMSETZUNGEN VON 2-ARYLMALEINSAEUREANHYDRIDEN MIT ARYLHYDRAZINEN" ZEITSCHRIFT FUER CHEMIE, DEUTSCHER VERLAG FUER GRUNDSTOFFINDUSTRIE,, DE, Bd. 13, Nr. 6, 1973, Seiten 214-216, XP000901338 ISSN: 0044-2402 in der Anmeldung erwähnt

## Beschreibung

Die vorliegende Erfindung betrifft neue agrochemische Zusammensetzungen mit fungizider Wirkung enthaltend Pyrrolidone als Wirkstoffe, sowie deren Verwendung bei der Behandlung von Pflanzen und in der Landwirtschaft.

Gegenstand der vorliegenden Erfindung sind Zusammensetzung enthaltend als Wirkstoffe Verbindungen der Formel I 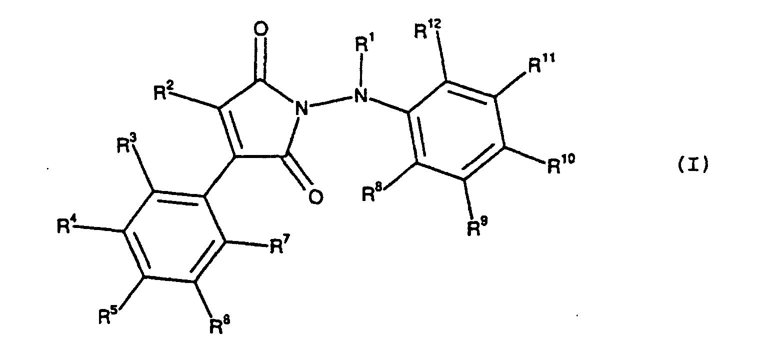 wobei die Reste folgende Bedeutungen haben:
- R¹: Wasserstoff, C₁-C₆-Alkyl, C₁-C₆-Alkylcarbonyl, Formyl oder C₁-C₆-Halogenalkylcarbonyl;
- R²: Halogen, C₁-C₆-Alkylthio, C₁-C₆-Alkoxy, C₃-C₆-Cycloalkyl-C₁-C₆-alkoxy, C₁-C₆-Alkoxy-C₁-C₆-alkyl, Halogen-C₁-C₆-alkoxy, C₁-C₆-Alkylsulfonyl, C₁-C₆-Alkylsulfinyl, Halogen-C₁-C₆-alkylsulfonyl, Cyano oder ein Rest NR¹³R¹⁴;
- R³ - R¹²: Wasserstoff, Halogen, C₁-C₈-Cycloalkyl, C₁-C₆-Alkyl, C₁-C₆-Halogenalkyl, C₁-C₆-Alkoxy, C₁-C₆-Halogenalkoxy, C₁-C₆-Alkylsulfonyl, C₁-C₆-Halogenalkylsulfonyl, Formyl, C₁-C₆-Alkylcarbonyl, Cyano, C₁-C₆-Alkylthio oder Phenyl, welches ggf. durch Halogenatome, C₁-C₆-Alkyl- oder C₁-C₆-Halogenalkyl-Gruppen substituiert sein kann,
- R¹³: Wasserstoff, C₁-C₆-Alkyl
- R¹⁴: C₁-C₆-Alkyl, C₁-C₈-Cycloalkyl oder gemeinsam mit R¹³ und dem Stickstoffatom, an das sie gebunden sind, einen gesättigten oder ungesätttigten heterocylischen fünf- oder sechsgliedrigen Ring bedeuten, der ein oder zwei Heteroatome ausgewählt aus der Gruppe Stickstoffatom oder Sauerstoffatom enthält,
sowie deren landwirtschaftlich einsetzbaren Salze.

Einige der Verbindungen der Formel I sind literaturbekannt. So werden beispielsweise in Z. Chem. Band 13, S. 214-216 (1973) (M. Augustin und P. Reinemann) phenyl-substituierte Pyrrolidone beschrieben. Eine fungizide Wirkung dieser Verbindungen ist bisher noch nicht beschrieben.

Die JP-A-50 117 929 beschreibt fungizide N-Anilino-3,4-dichlor - 3-pyrrolin-2,5-dione. Außer der Dichlorsubstitution in 3- und 4-Stellung wird keine weitere Substitutionsmöglichkeit beschrieben oder nahegelegt.

Überraschenderweise wurde gefunden, daß Verbindungen der Formel I eine bemerkenswerte fungizide Wirkung aufweisen. Sie eignen sich zur Bekämpfung von Schadpilzen bei der Behandlung von Pflanzen, als auch zur therapeutischen Behandlung von durch Schadpilzen verursachten Erkrankungen am Menschen, sowie zur veterinärischen Behandlung bei Säugetieren.

Verbindungen der Formel I lassen sich analog zu dem in der Literatur (Z. Chem. Band 13, S. 214.216 (1973)) beschriebenen Verfahren herstellen. Die Ausgangsstoffe sind entweder literaturbekannt oder kommerziell erhältlich.

Bei der Definition der Substituenten R¹ bis R¹² stehen die angegebenen Begriffe als Sammelbegriff für eine Gruppe von Verbindungen. Die jeweils genannten Alkylreste stehen jeweils für geradkettige oder verzweigte Alkylreste mit bis zu sechs C-Atomen.

Halogen steht jeweils für Fluor, Brom, Chlor oder Iod, insbesondere für Fluor oder Chlor.

Ferner stehen beispielsweise:
- C₁-C₆-Alkyl für eine geradkettige oder verzweigte Alkylgruppe, wie z.B. Methyl, Ethyl, n-Propyl, 1-Methylethyl, n-Butyl, 1-Methylpropyl, 2-Methylpropyl oder 1,1-Dimethylethyl, insbesondere für Ethyl;
- C₁-C₆-Halogenalkyl für einen C₁-C₆-Alkylrest wie vorstehend genannt, der partiell oder vollständig, insbesondere ein-, zwei- oder dreifach, durch Fluor, Chlor, Brom und/oder Iod substituiert ist, z.B. Trichlormethyl, Trifluormethyl, 2-Fluorethyl, 2-Chlorethyl, 2-Bromethyl, 2,2-Difluorethyl, 2,2,2-Trifluorethyl, 2,2,2-Trichlorethyl, 2-Fluorpropyl, 3-Fluorpropyl, 2-Chlorpropyl oder 3-Chlorpropyl, insbesondere für 2-Fluorethyl oder 2-Chlorethyl;
- C₁-C₆-Alkoxy für einen geradkettigen oder verzweigten Alkoxyrest mit bis zu sechs C-Atomen, wie z.B. Methoxy, Ethoxy, n-Propoxy, 1-Methylethoxy, n-Butoxy, 1-Methylpropoxy, 2-Methylpropoxy oder 1,1-Dimethylethoxy, insbesondere für Methoxy oder Ethoxy;
- C₁-C₆-Alkoxy-C₁-C₆-alkyl für einen Alkylrest, wie vorstehend genannt, der durch C₁-C₆-Alkoxy, wie vorstehend genannt, substituiert ist, wie z.B. Methoxymethyl, Ethoxymethyl, n-Propoxymethyl, 1-Methylethoxymethyl oder n-Butoxymethyl;
- C₃-C₈-Cycloalkyl für eine gesättigte Cycloalkylgruppe, wie z.B. Cyclopropyl, Cyclobutyl, Cyclopentyl, Cyclohexyl, Cycloheptyl, Cyclooctyl;
- C₃-C₈-Cycloalkyl-C₁-C₆-alkoxy für eine Alkoxygruppe, wie vorstehend genannt, die durch C₃-C₈-Cycloalkyl, wie vorstehend genannt, substituiert ist; wie z.B. Cyclopropylmethoxy, Cyclobutylmethoxy, Cyclopentylmethoxy, Cyclohexylmethoxy, Cycloheptylmethoxy, Cyclooctylmethoxy, Cyclopropylethoxy, Cyclobutylethoxy, Cyclopentylethoxy, Cyclohexylethoxy, Cycloheptylethoxy, Cyclooctylethoxy;
- Halogen-C₁-C₆-alkoxy für einen C₁-C₆-Alkoxyrest, wie vorstehend genannt, der durch Fluor, Chlor oder Brom ein-, zweioder dreifach substituiert ist, wie z.B. Chlormethoxy, Fluormethoxy, Difluormethoxy, Difluorethoxy, Dichlormethoxy, Dichlorethoxy;
- C₁-C₆-Alkyl-carbonyl für eine Carbonylgruppe, die durch einen C₁-C₆-Alkylrest wie vorstehend substituiert ist, wie z.B. Acetyl, Propionyl, Butyryl;
- Halogen-C₁-C₆-Alkyl-carbonyl für: einen C₁-C₆-Alkyl-carbonylrest wie vorstehend, der durch Fluor, Chlor oder Brom substituiert ist;
- C₁-C₆-Alkylsulfonyl für eine Sulfonylgruppe, die durch einen C₁-C₆-Alkylrest, wie vorstehend genannt, substituiert ist;
- C₁-C₆-Alkylsulfinyl für eine Sulfinylgruppe, die durch einen C₁-C₆-Alkylrest, wie vorstehend genannt, substituiert ist;
- Halogen-C₁-C₆-alkylsulfonyl für: einen C₁-C₆-Alkylsulfonylrest wie vorstehend genannt, der durch Fluor, Chlor oder Brom substituiert ist;
- C₁-C₆-Alkylthio für: ein Schwefelatom, das durch einen C₁-C₆-Alkylrest wie vorstehend genannt substituiert ist;
- ein gegebenenfalls substituierter Phenylrest: ein Phenylrest, der unsubstituiert oder ein- oder mehrfach substituiert ist. Die Substituenten sind beliebig, beispielsweise folgende: Halogenatome, C₁-C₆-Alkyl oder Halogen-C₁-C₆-Alkyl. Der Phenylrest ist vorzugsweise ein-, zwei- oder dreifach substituiert.

Für den Fall, daß R¹³ und R¹⁴ zusammen mit dem N-Atom, an das sie gebunden sind, gemeinsam eine Kette von 4-5 C-Atomen bilden, handelt es sich um gesättigte oder teilweise ungesättigte heterocylische fünf- oder sechsgliedrige Ringe, die ein oder zwei Heteroatome (Sauerstoff- oder Stickstoffatome) enthalten, wie z.B. Pyrrol, Oxazol, Isoxazol, Morpholino oder Piperidino.

Folgende Verbindungen kommen im Sinne der vorliegenden Erfindung bevorzugt im Hinblick auf die genannten ubstituentendefinitionen, jeweils für sich alleine oder in Kombination miteinander, in Frage:
1. Verbindungen der Formel I, wobei R¹ die folgenden Bedeutungen hat: Wasserstoff; C₁-C₃-Alkyl (wie z.B. Methyl, Ethyl); C₁-C₃-Alkylcarbonyl (z.B. Acetyl); Formyl; insbesondere Wasserstoff, Formyl, Acetyl oder Methyl.
2. Verbindungen nach Punkt 1, wobei R² die folgenden Bedeutungen (z.B. Methylsulfinyl); C₁-C₃-Halogenalkoxy (z.B. Difluormethoxy); insbesondere Chlor und Brom.
3. Verbindungen nach den Punkten 1 oder 2, wobei R³-R¹² die folgenden Bedeutungen haben: Wasserstoff; Fluor; Chlor; C₁-C₄-Alkyl (z.B. Methyl, Ethyl, Propyl, Butyl); Halogen-C₁-C₃-alkyl (z.B. Trifluormethyl, Difluormethyl); Halogen-C₁-C₃-alkoxy (z.B. Trifluormethoxy, Difluormethoxy); C₁-C₃-Alkoxy (z.B. Methoxy); C₁-C₃-Alkylthio (z.B. Methylthio); Cyano.
4. Verbindungen nach den Punkten 1 bis 3, wobei mindestens zwei der Reste R⁸-R¹² sowie außerdem mindestens zwei der Reste R³-R⁷ Wasserstoff bedeuten und die übrigen für Wasserstoff, Fluor, Chlor; C₁-C₄-Alkyl (z.B. Methyl, Ethyl, Propyl, Butyl); Halogen-C₁-C₃-alkyl (z.B. Trifluormethyl); Halogen-C₁-C₃-alkoxy (z.B. Trifluormethoxy, Difluormethoxy) stehen.

Die beiden Phenylringe sind bevorzugt unsubstituiert (R³ - R¹² = H) oder bevorzugt ein-, zwei- oder dreifach substituiert, wobei vorwiegend folgende Substituenten in Frage kommen: C₁-C₆-Alkyl, Halogen, Halogen-C₁-C₆-alkyl, Halogen-C₁-C₆-alkoxy. Besonders bevorzugt sind in diesem Sinne die folgenden Substituenten: Methyl, iso-Propyl, Fluor, Chlor, Trifluormethyl oder Trifluormethoxy.

Die zuvor genannten Verbindungen haben sich als in der Regel besonders wirksam erwiesen.

Im Sinne der vorliegenden Erfindung kommen beispielsweise folgende Verbindungen in Tabelle 1 als fungizide Wirkstoffe in Frage:

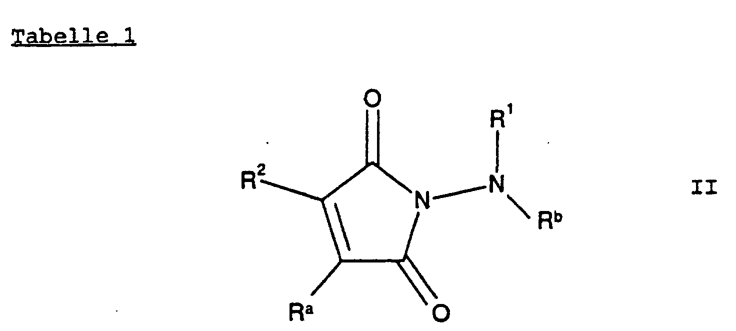

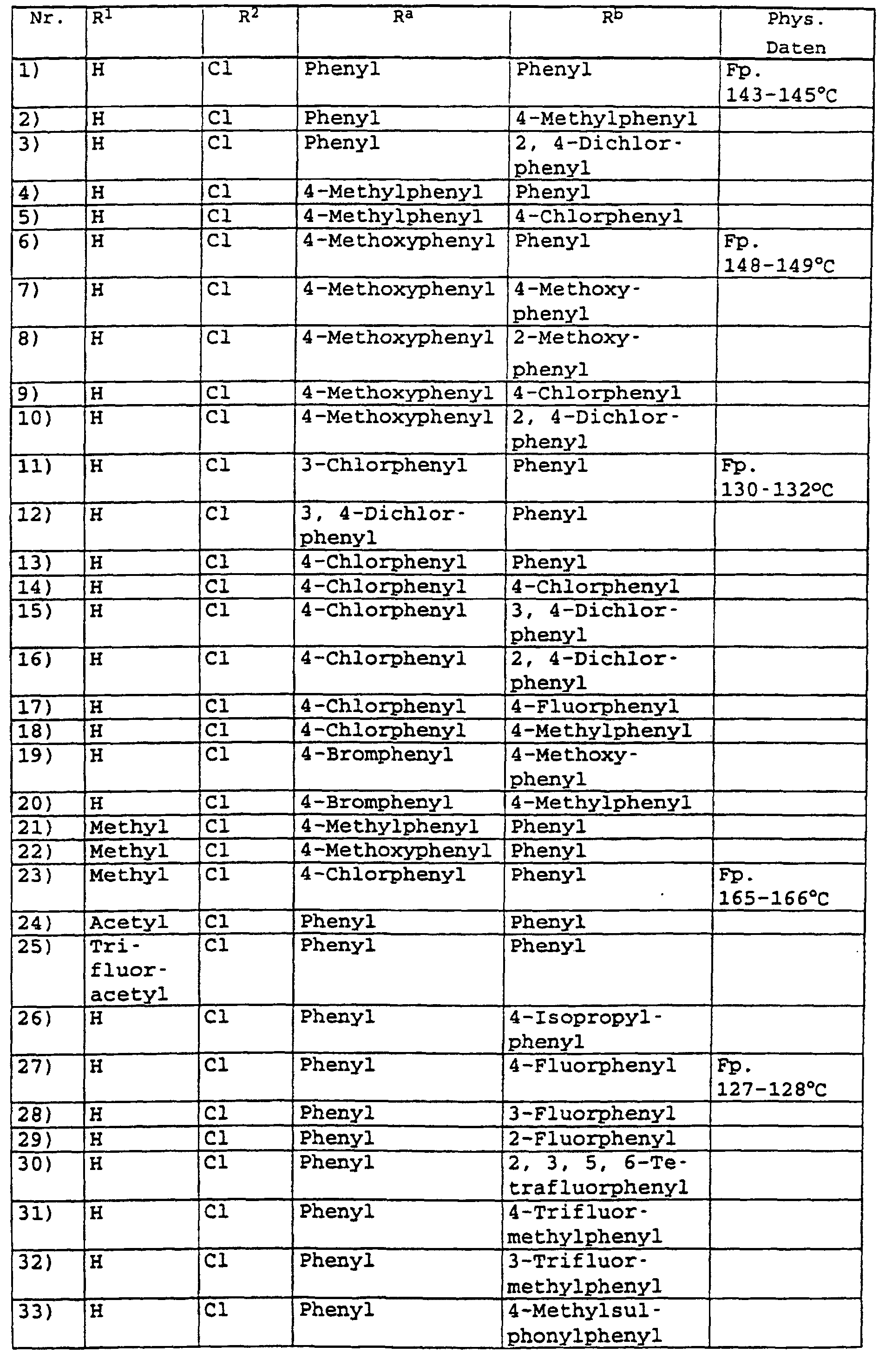

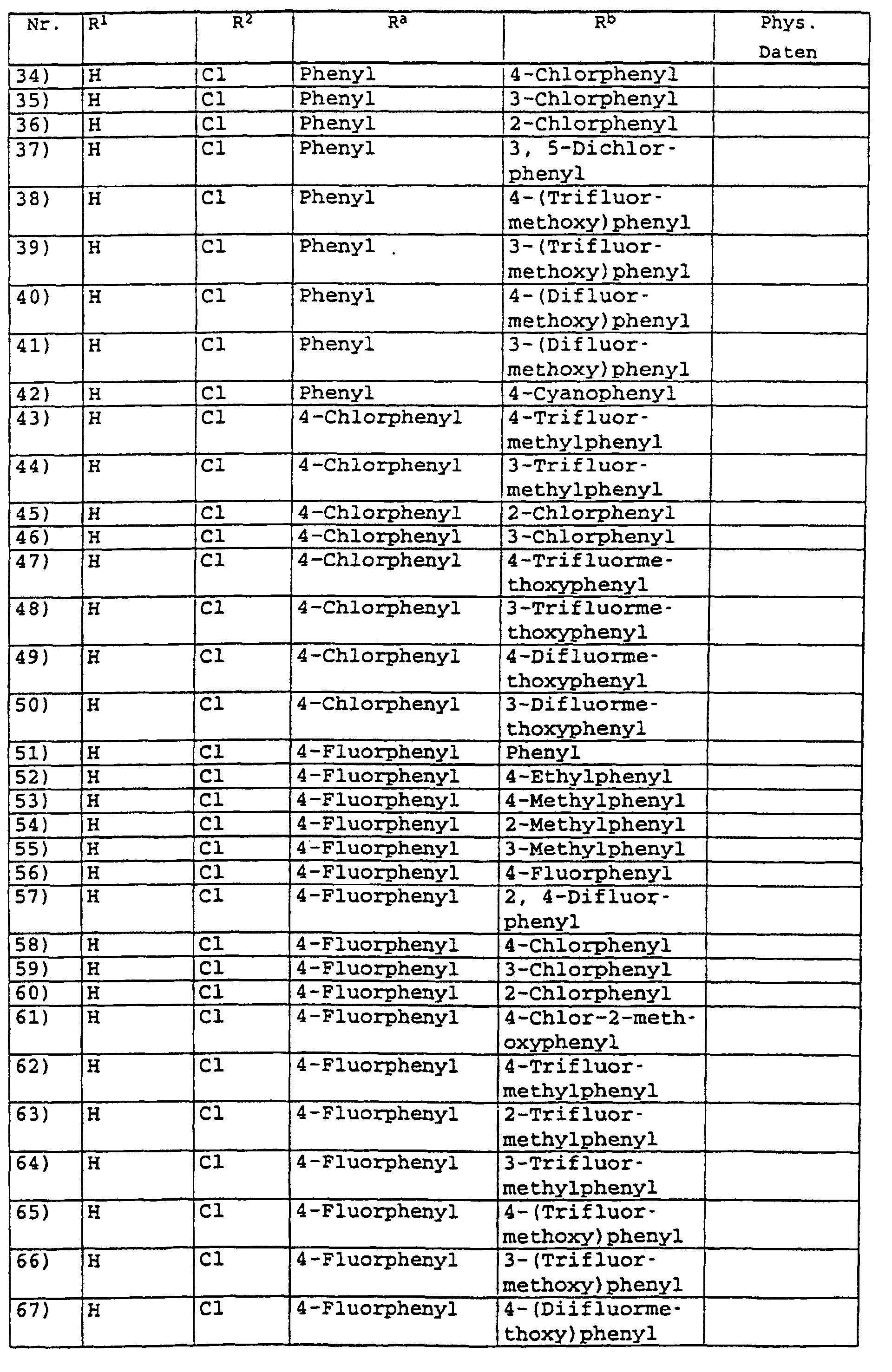

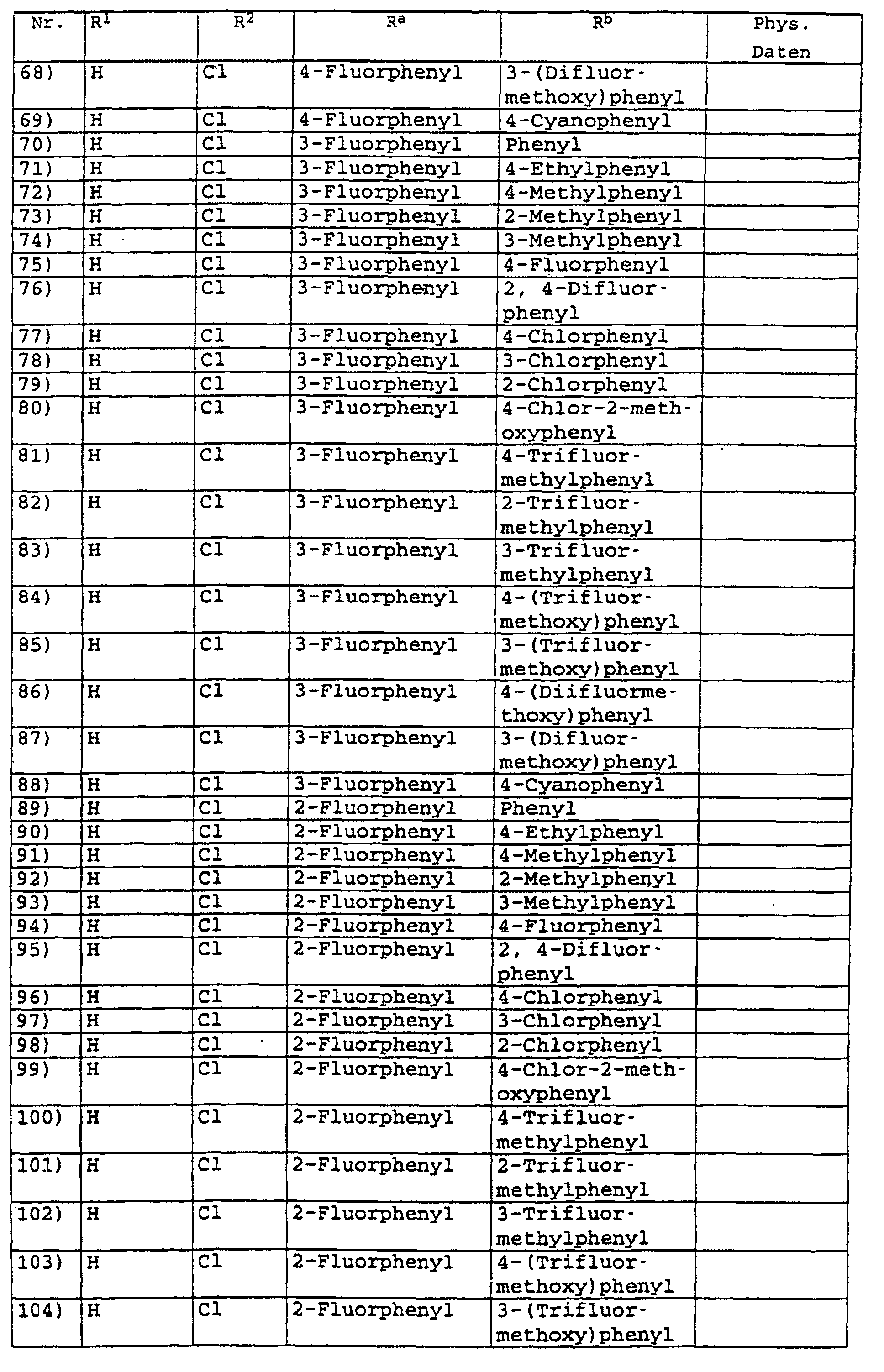

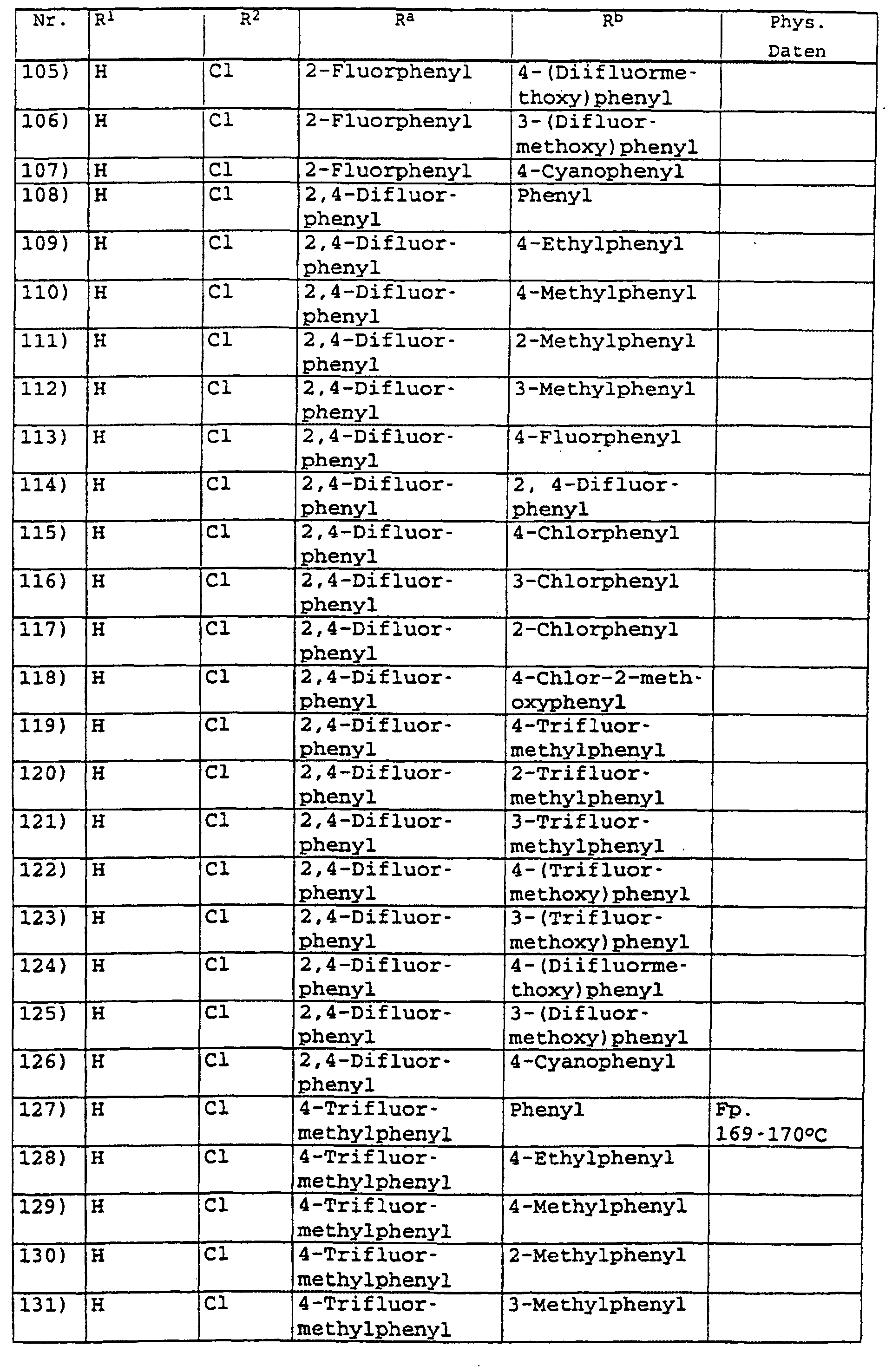

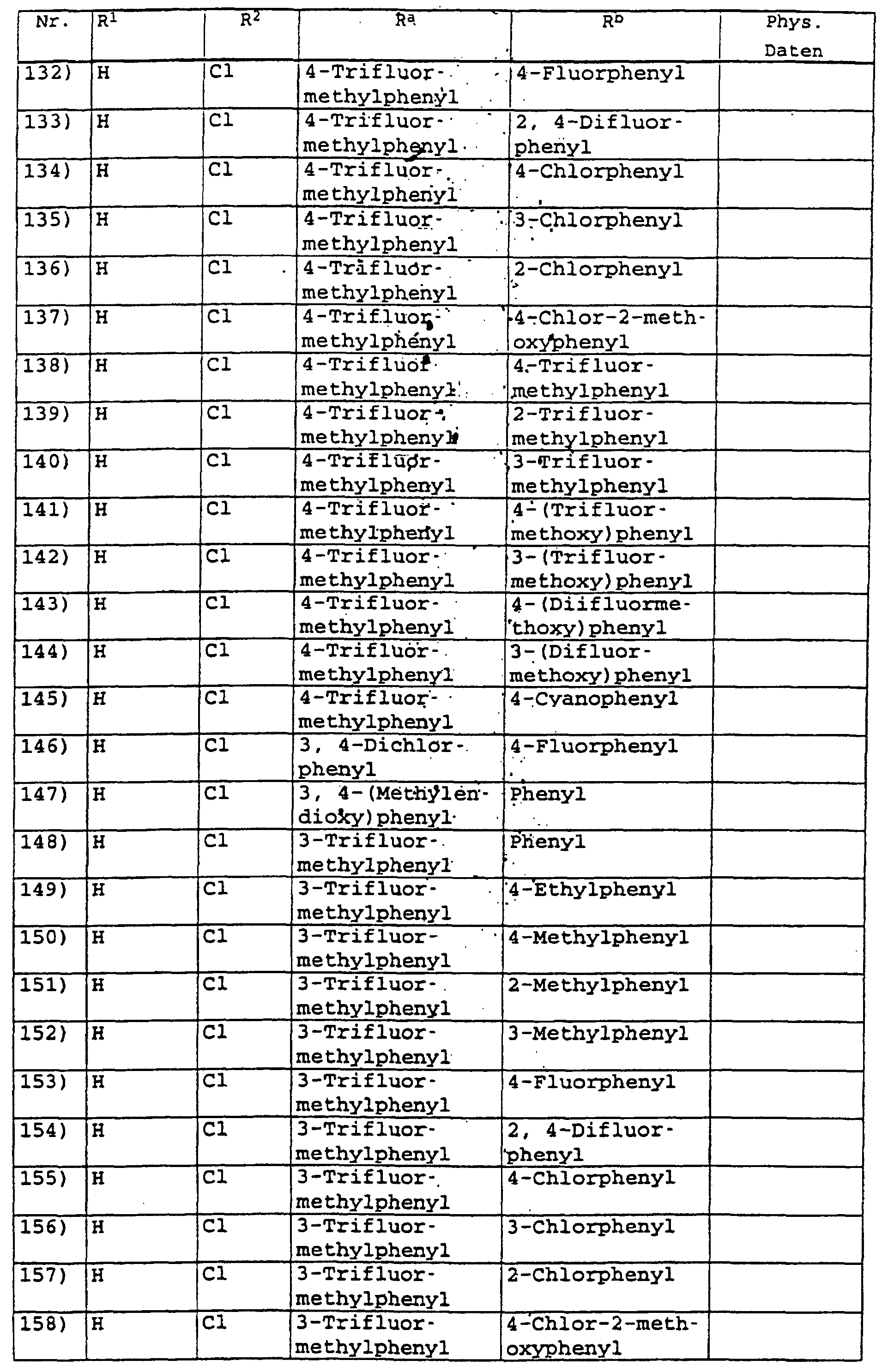

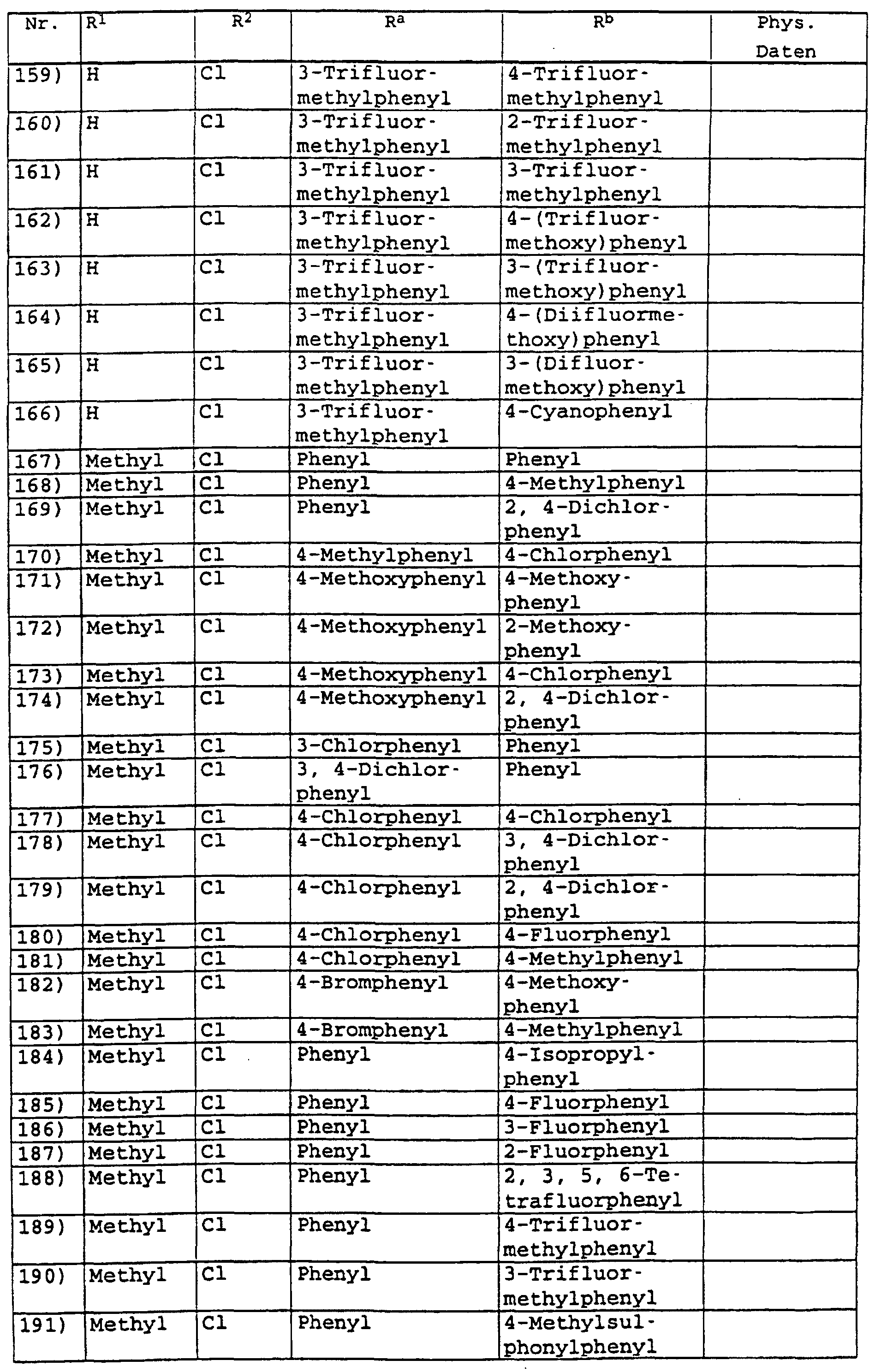

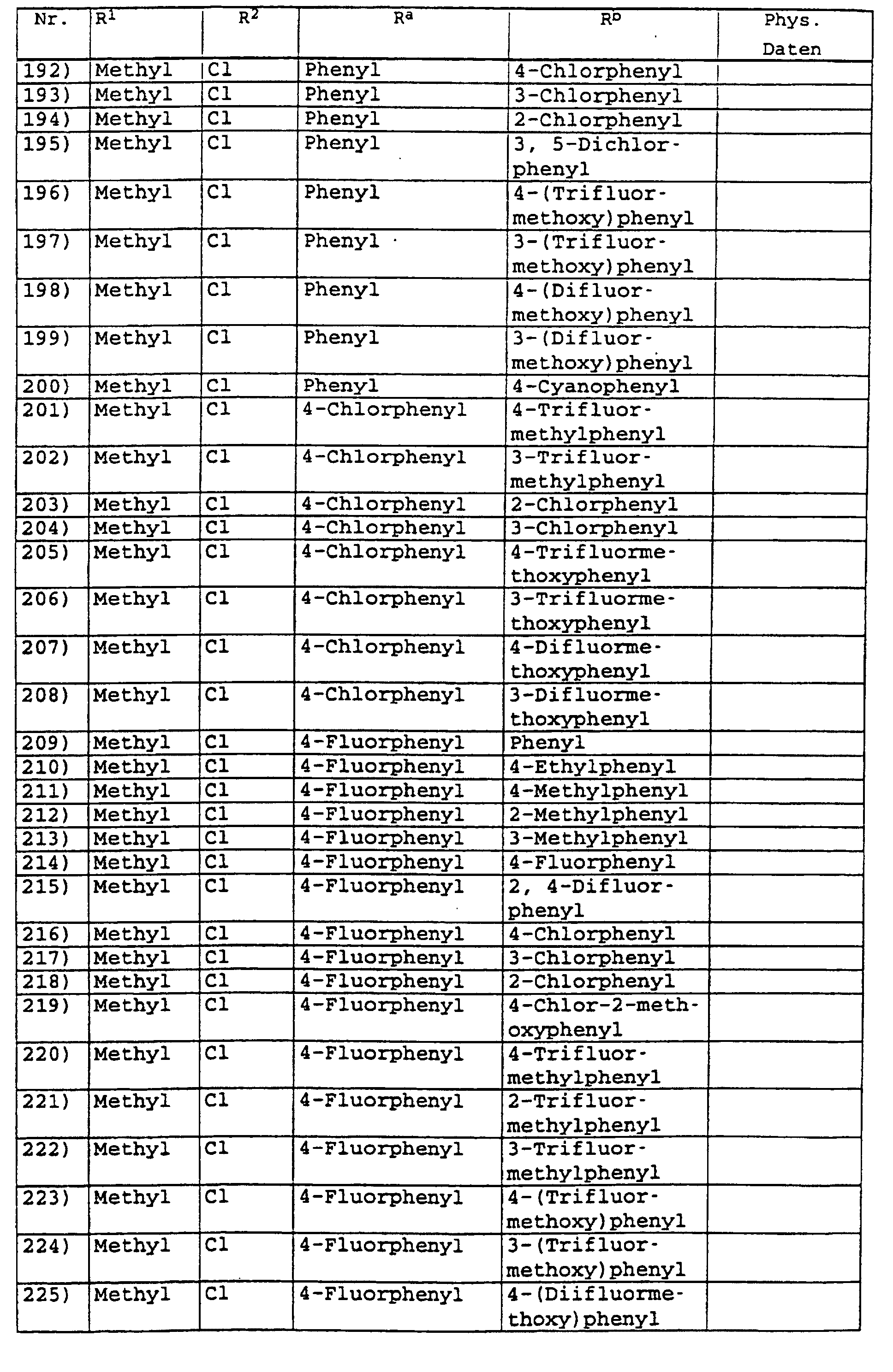

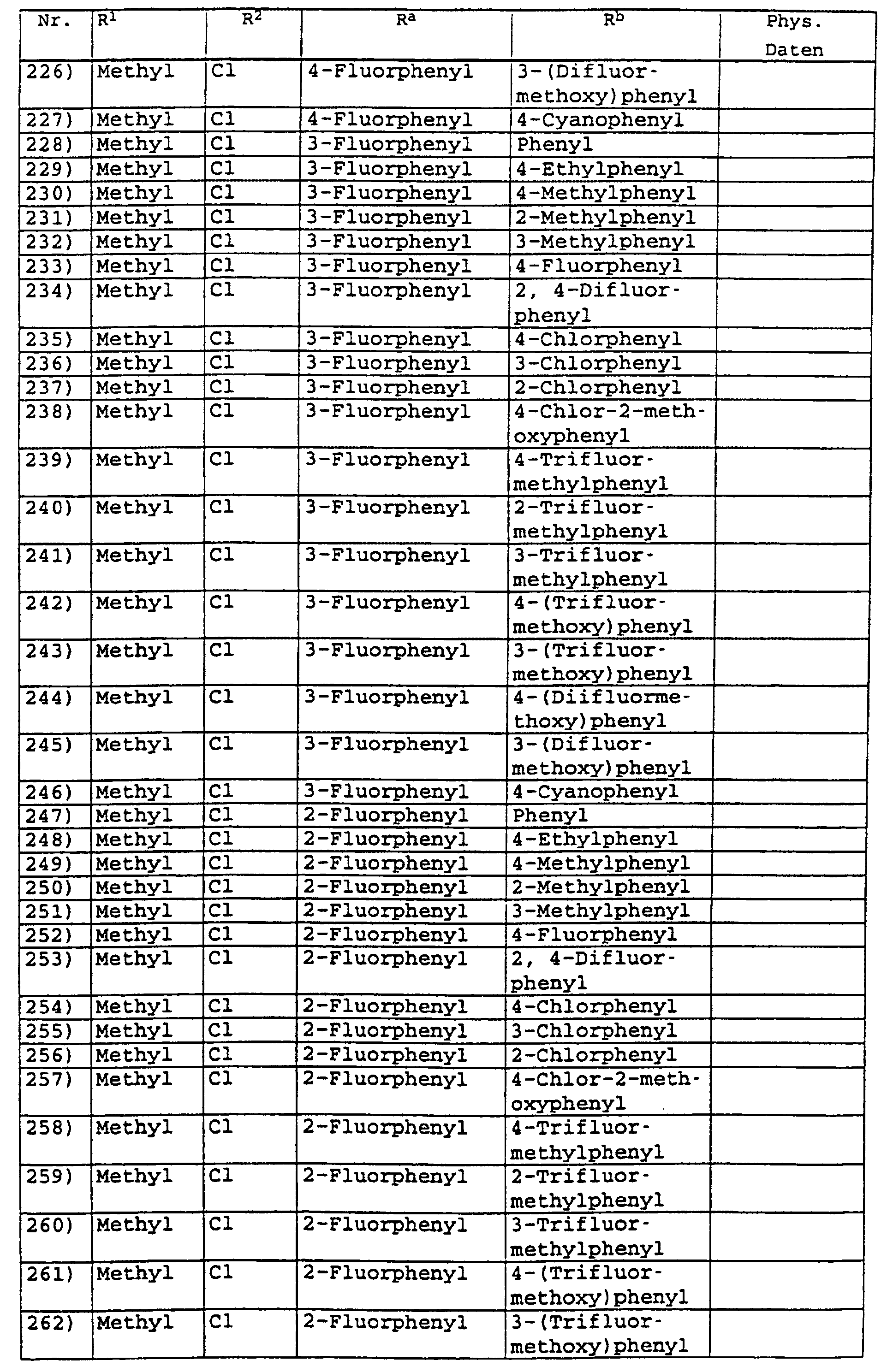

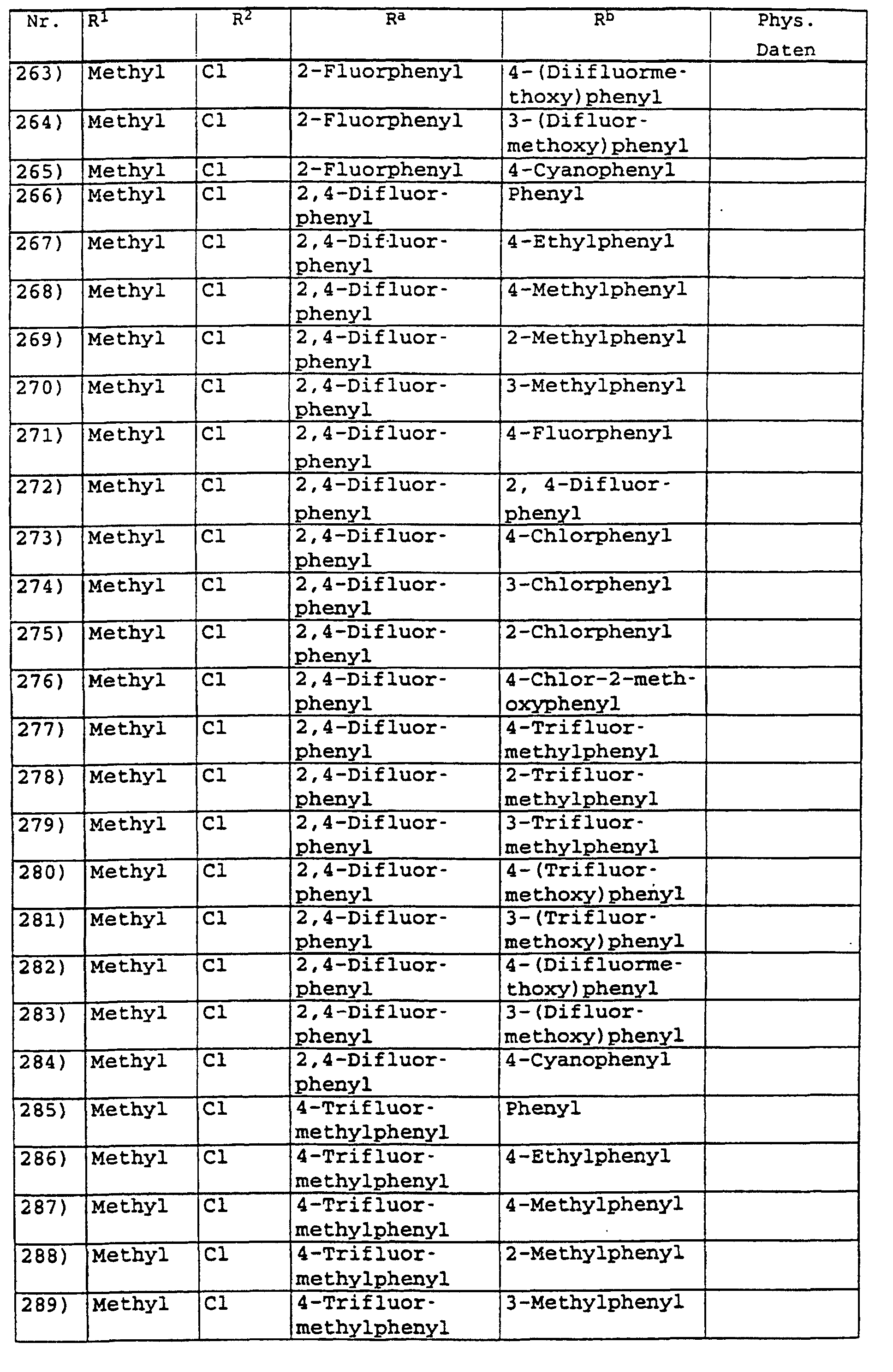

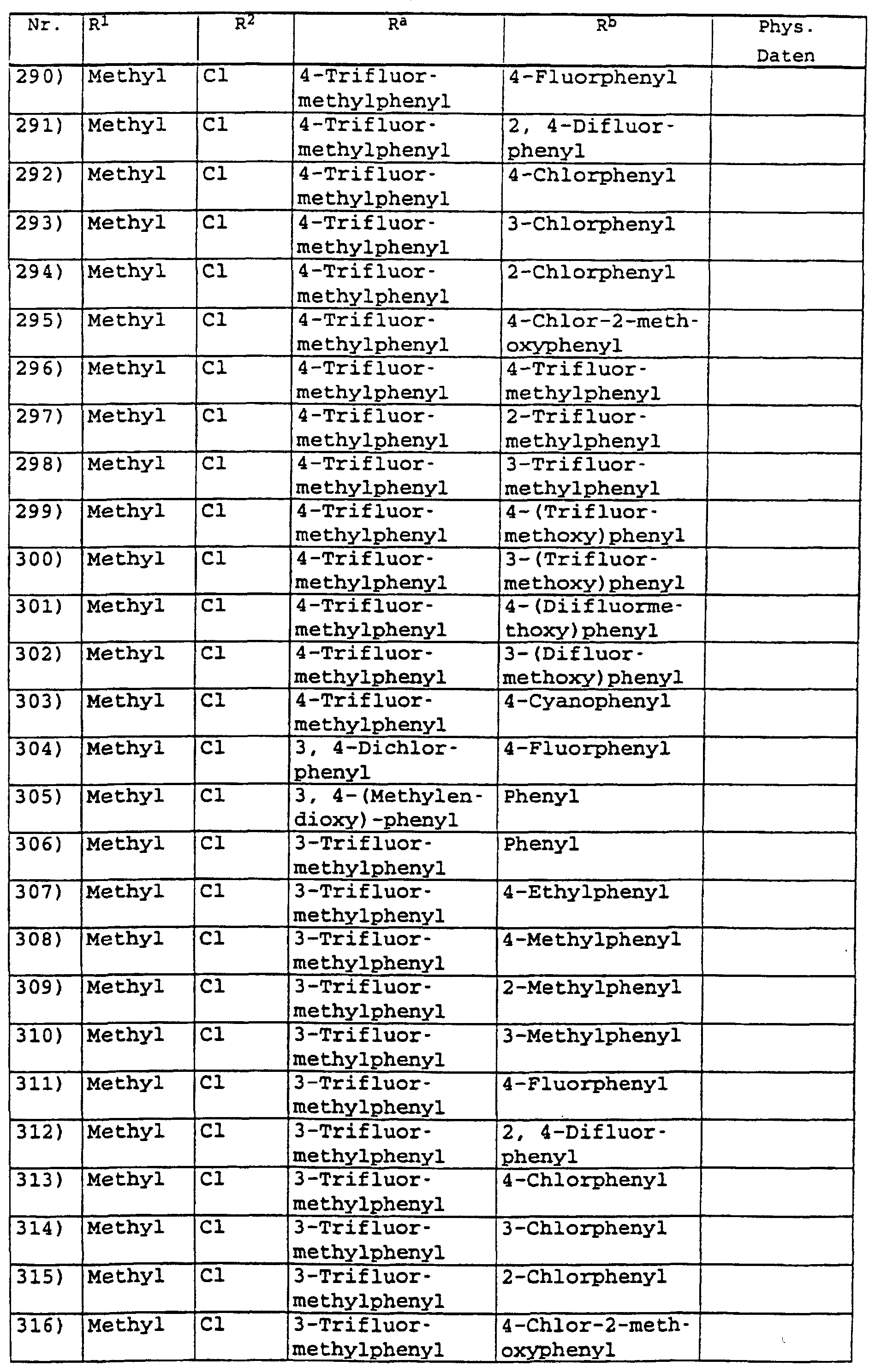

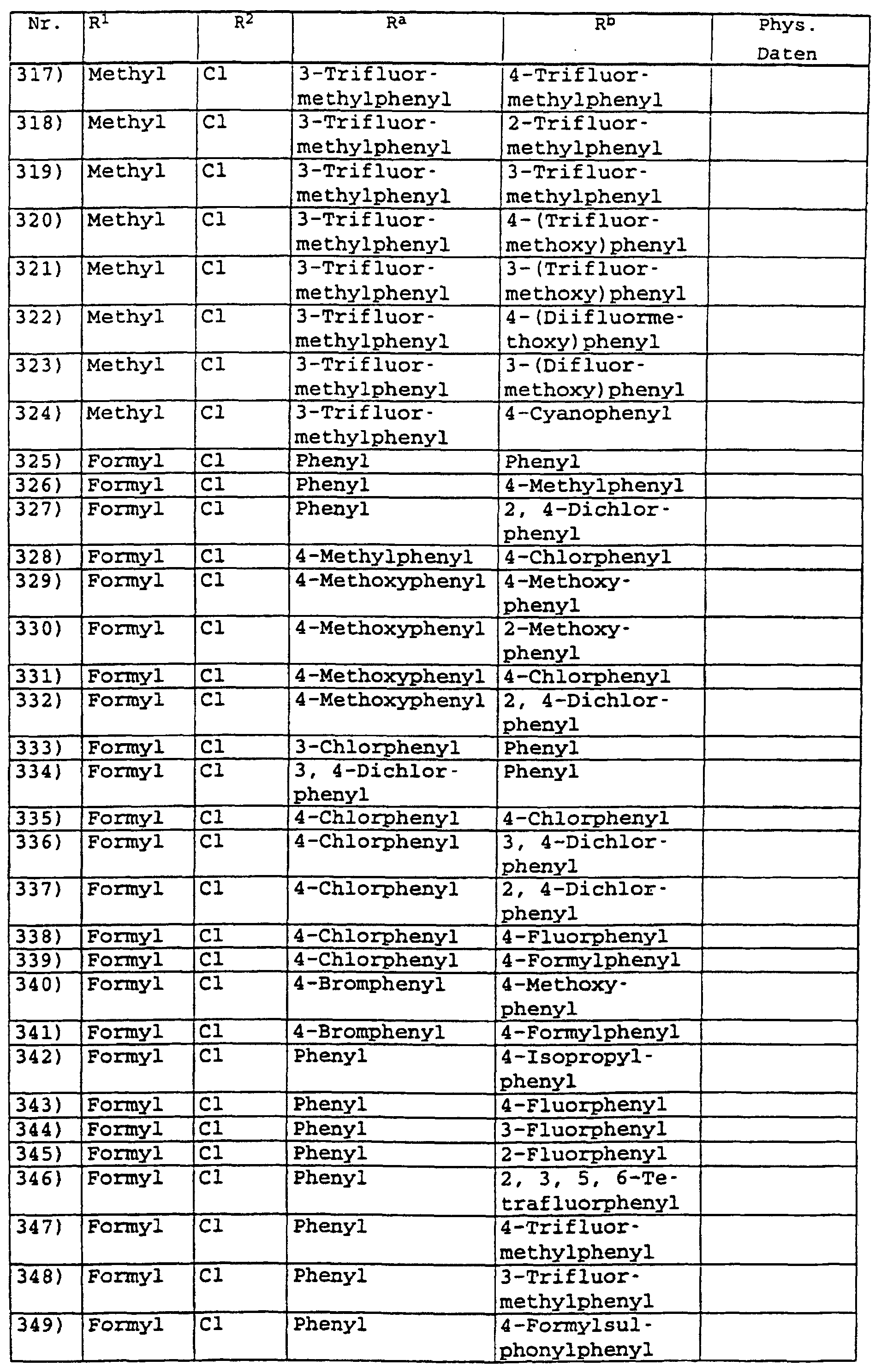

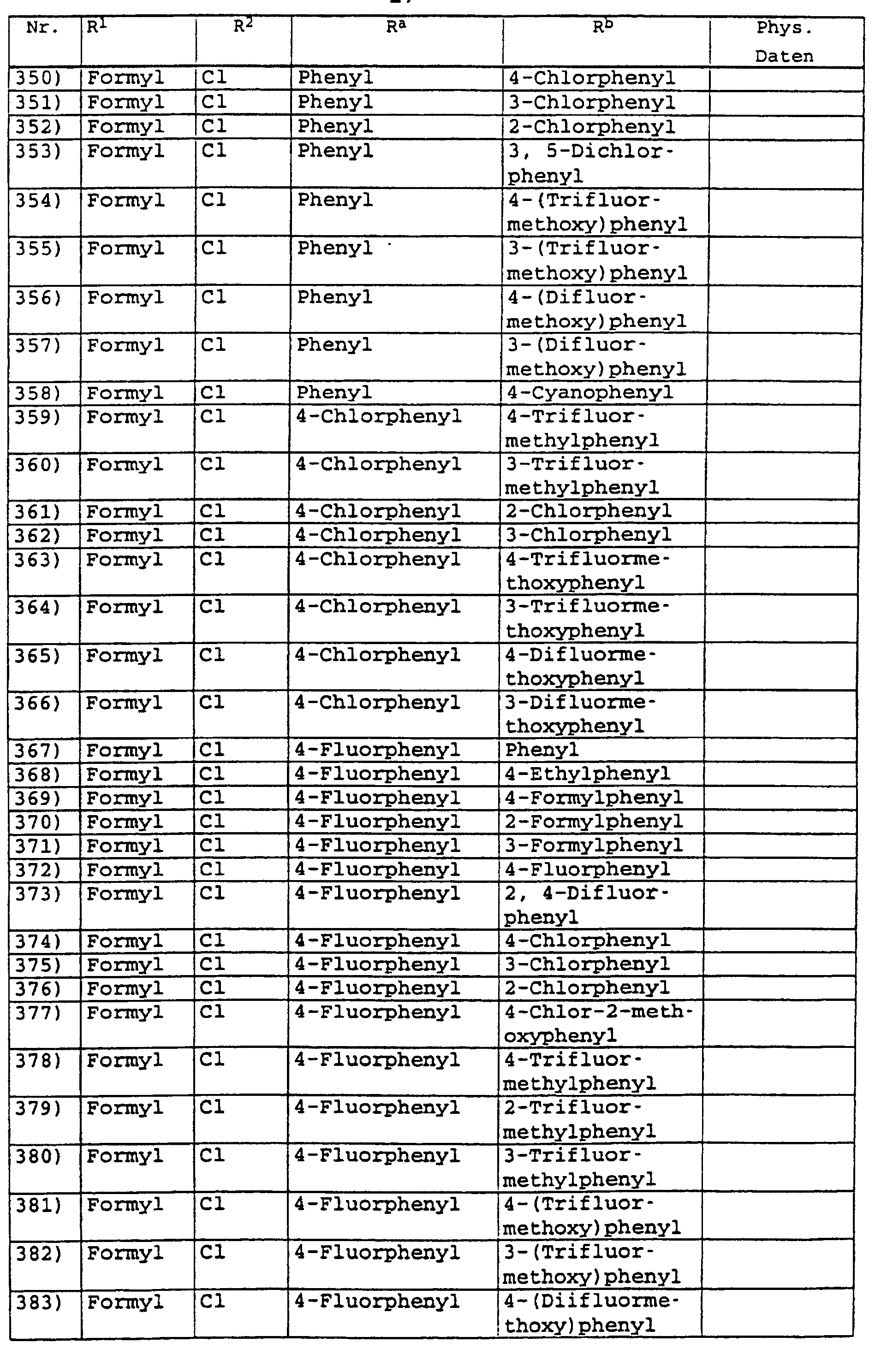

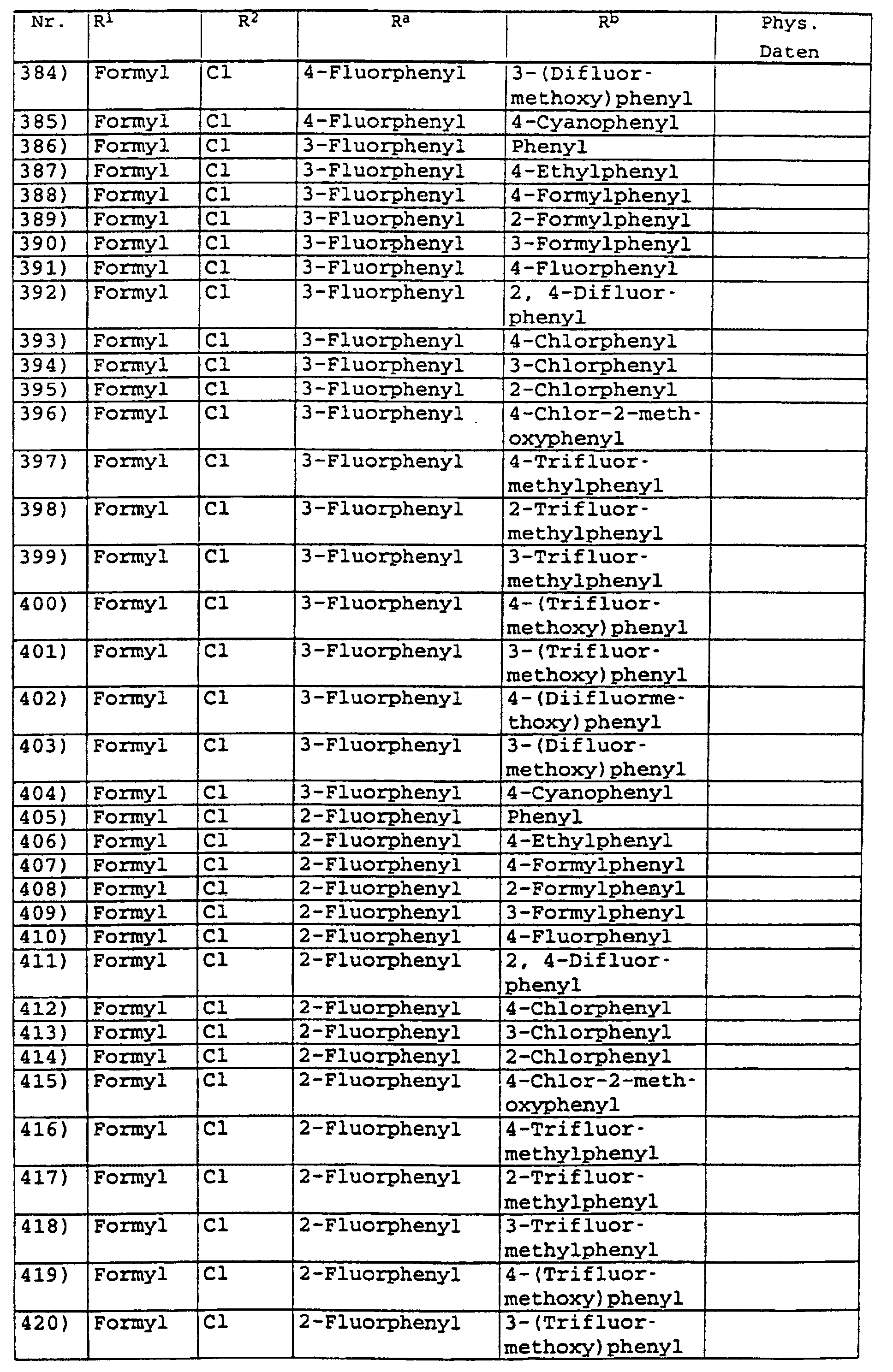

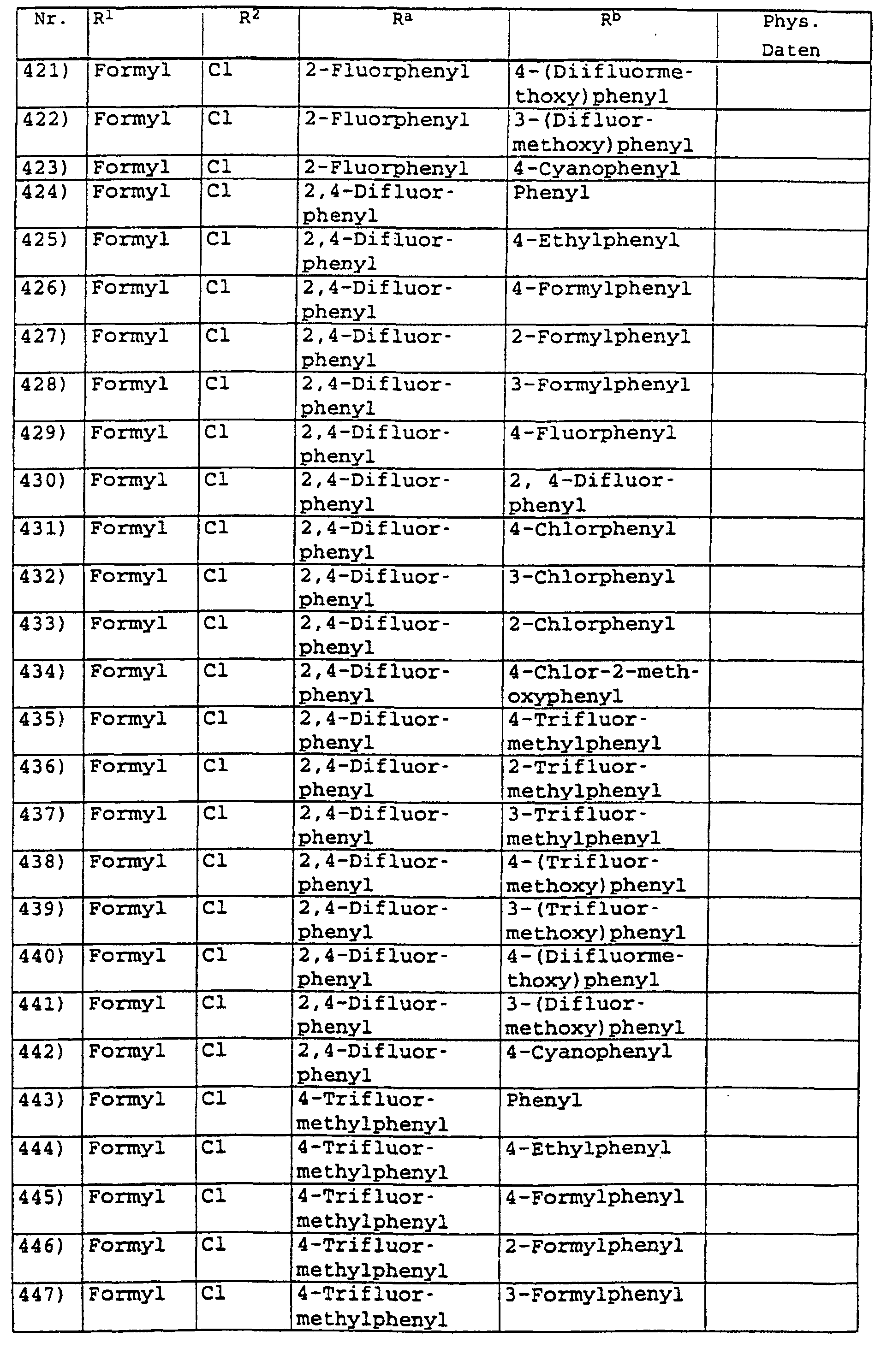

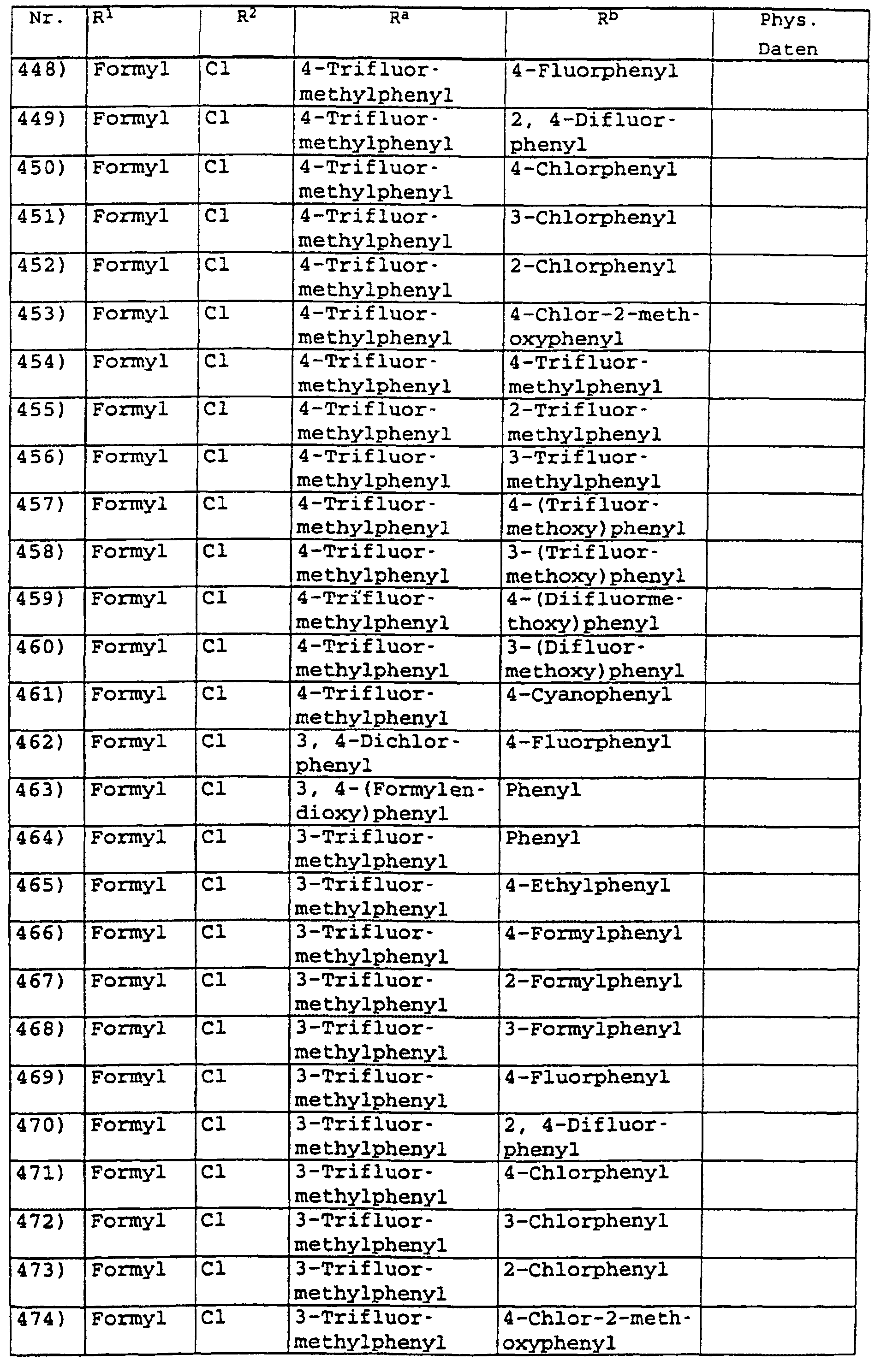

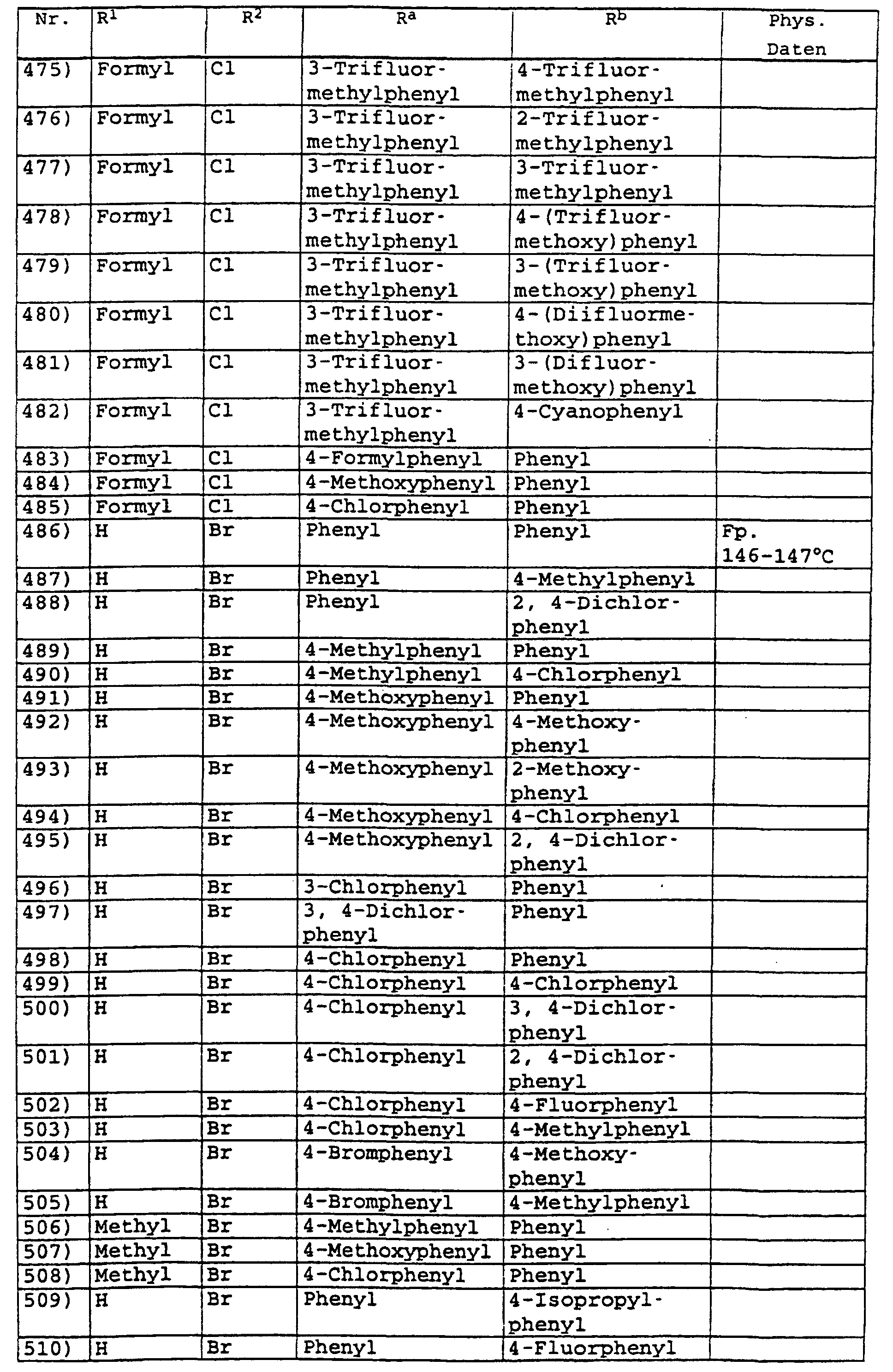

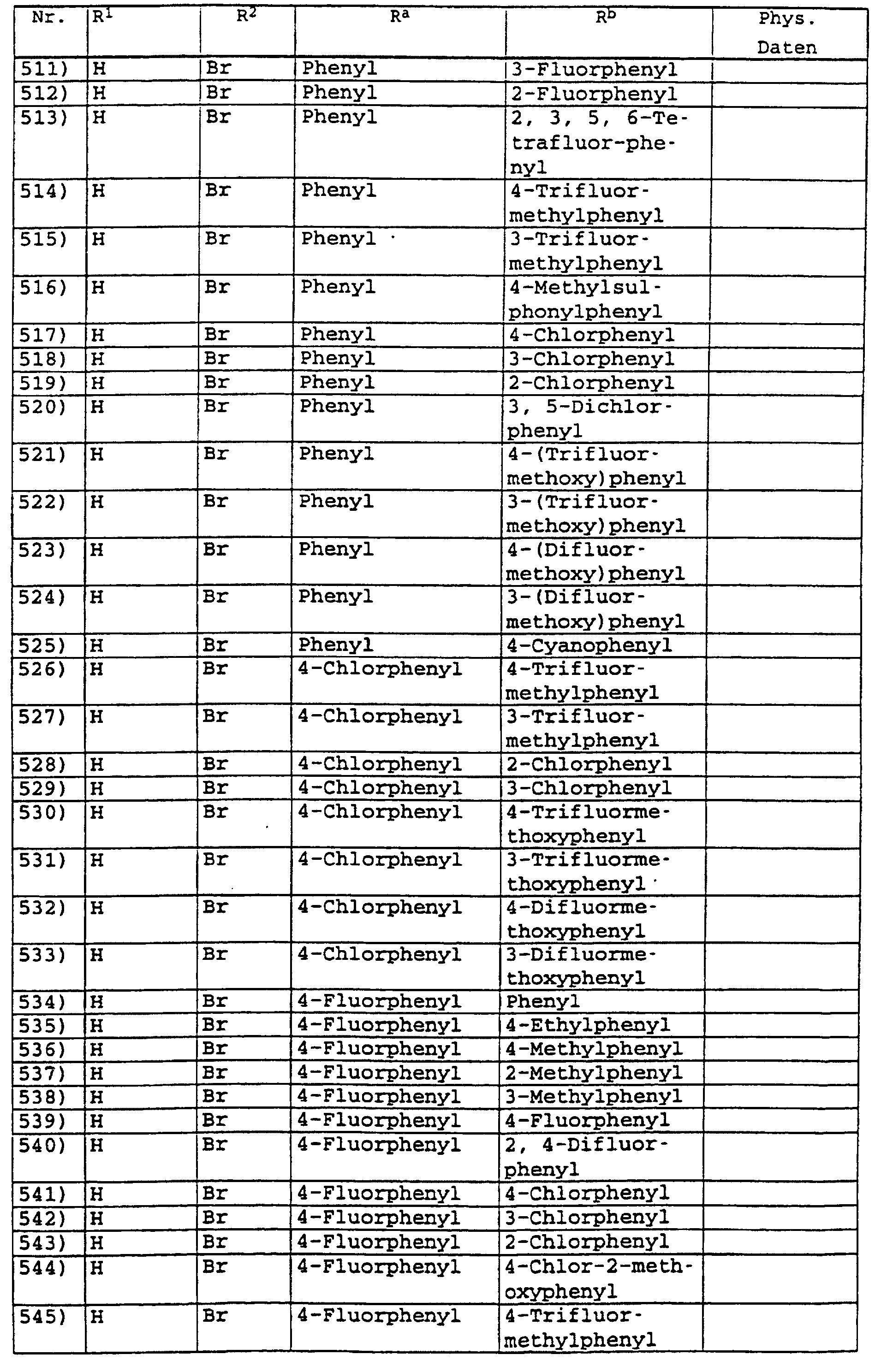

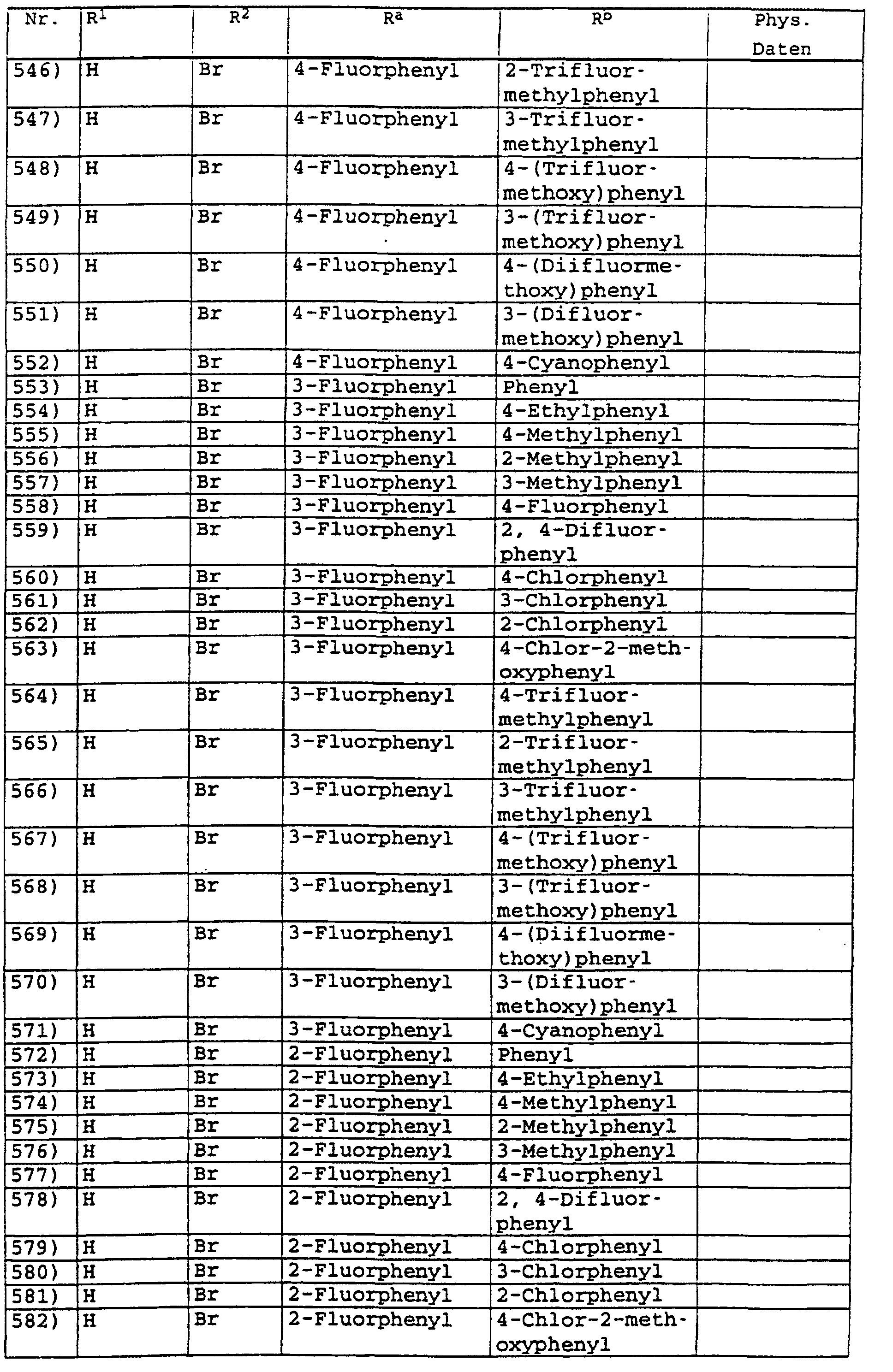

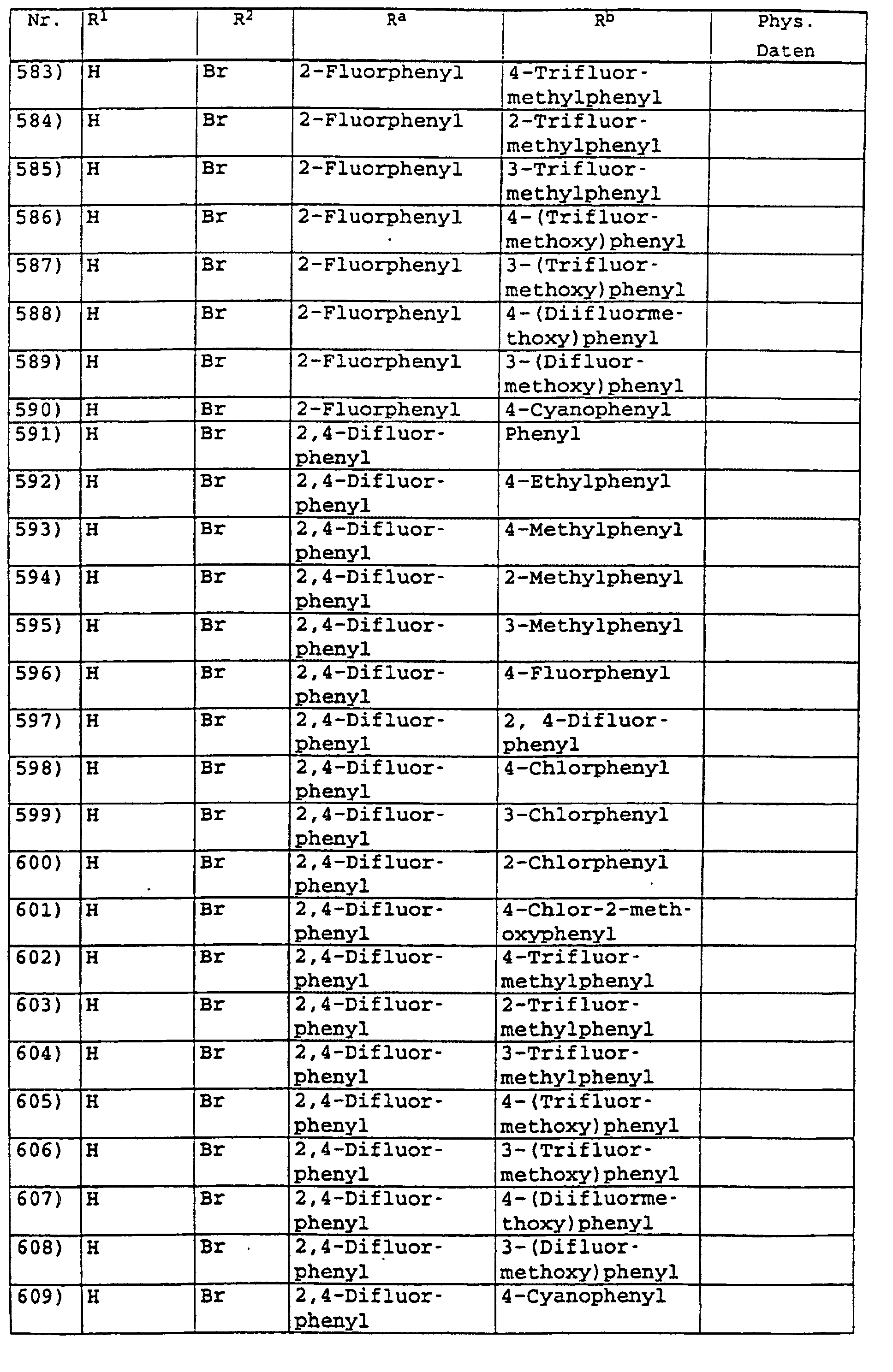

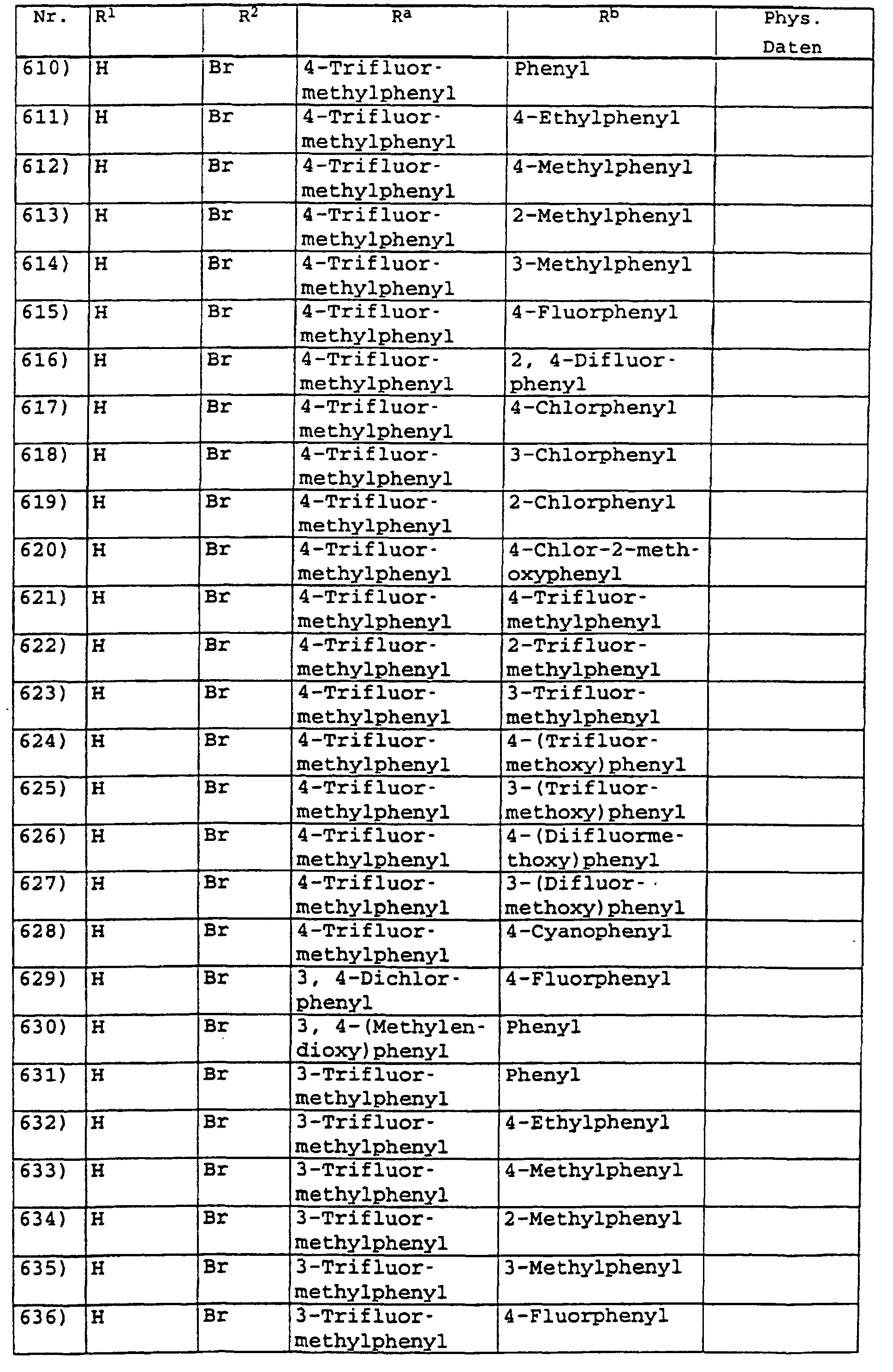

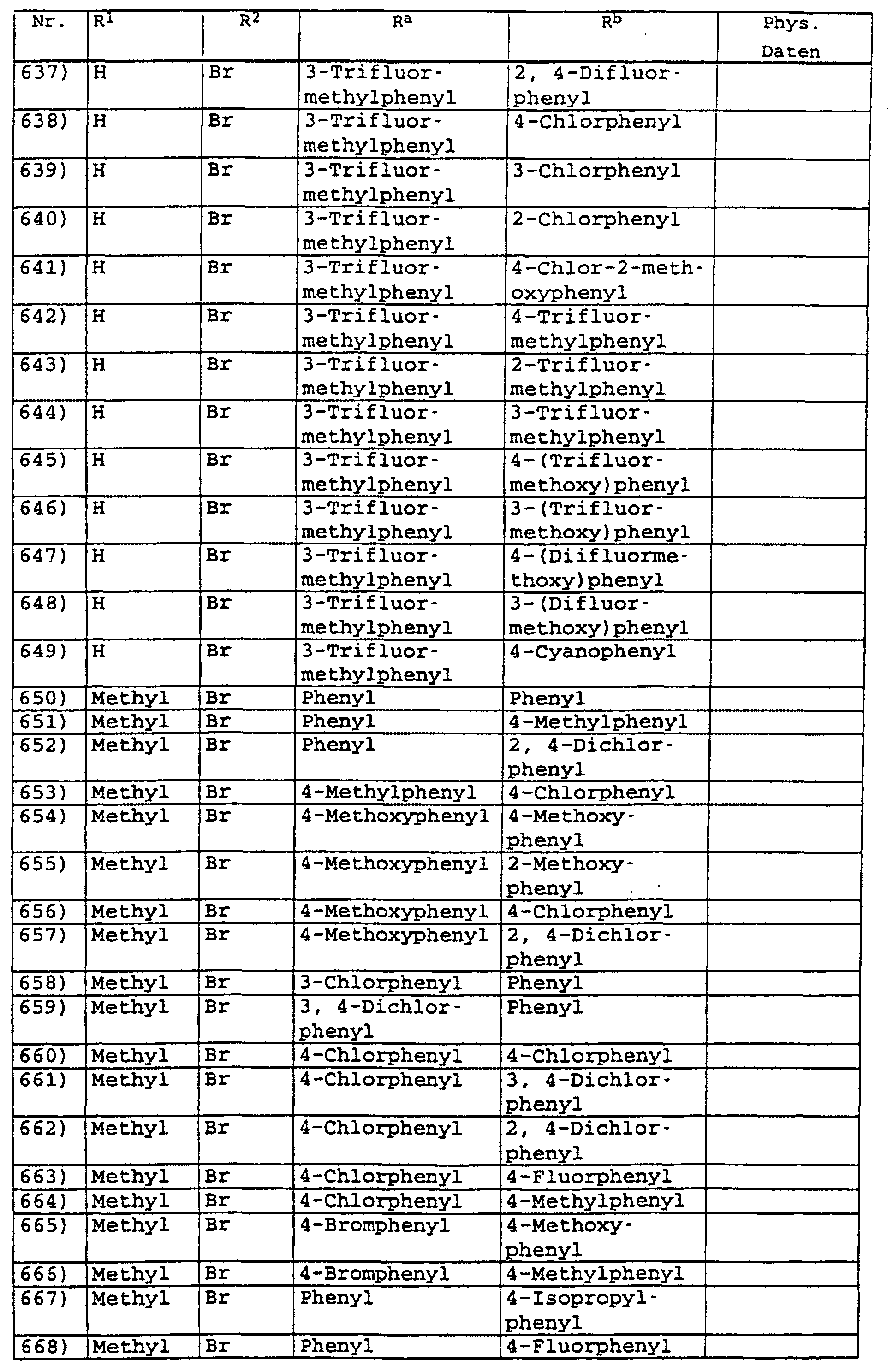

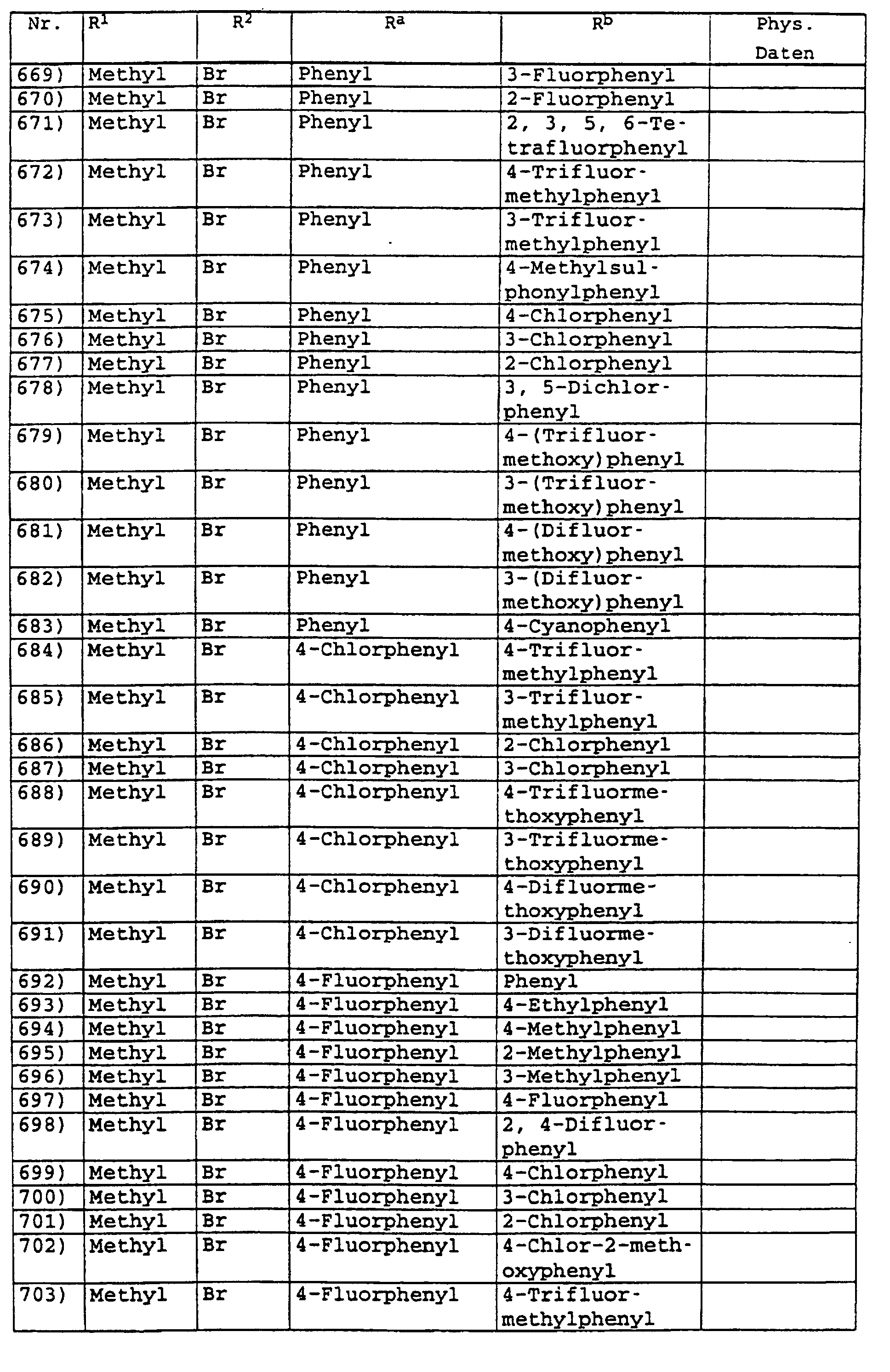

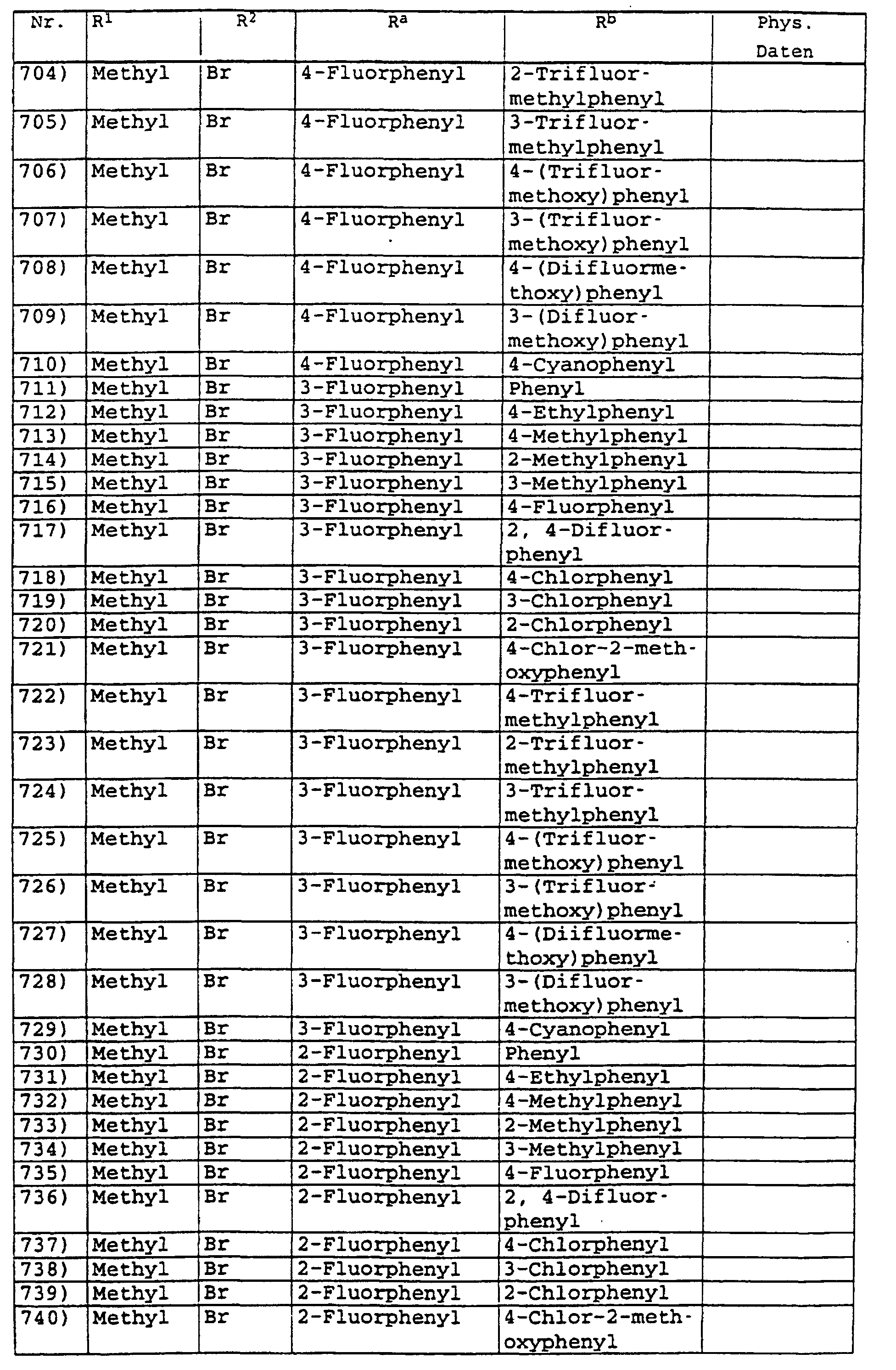

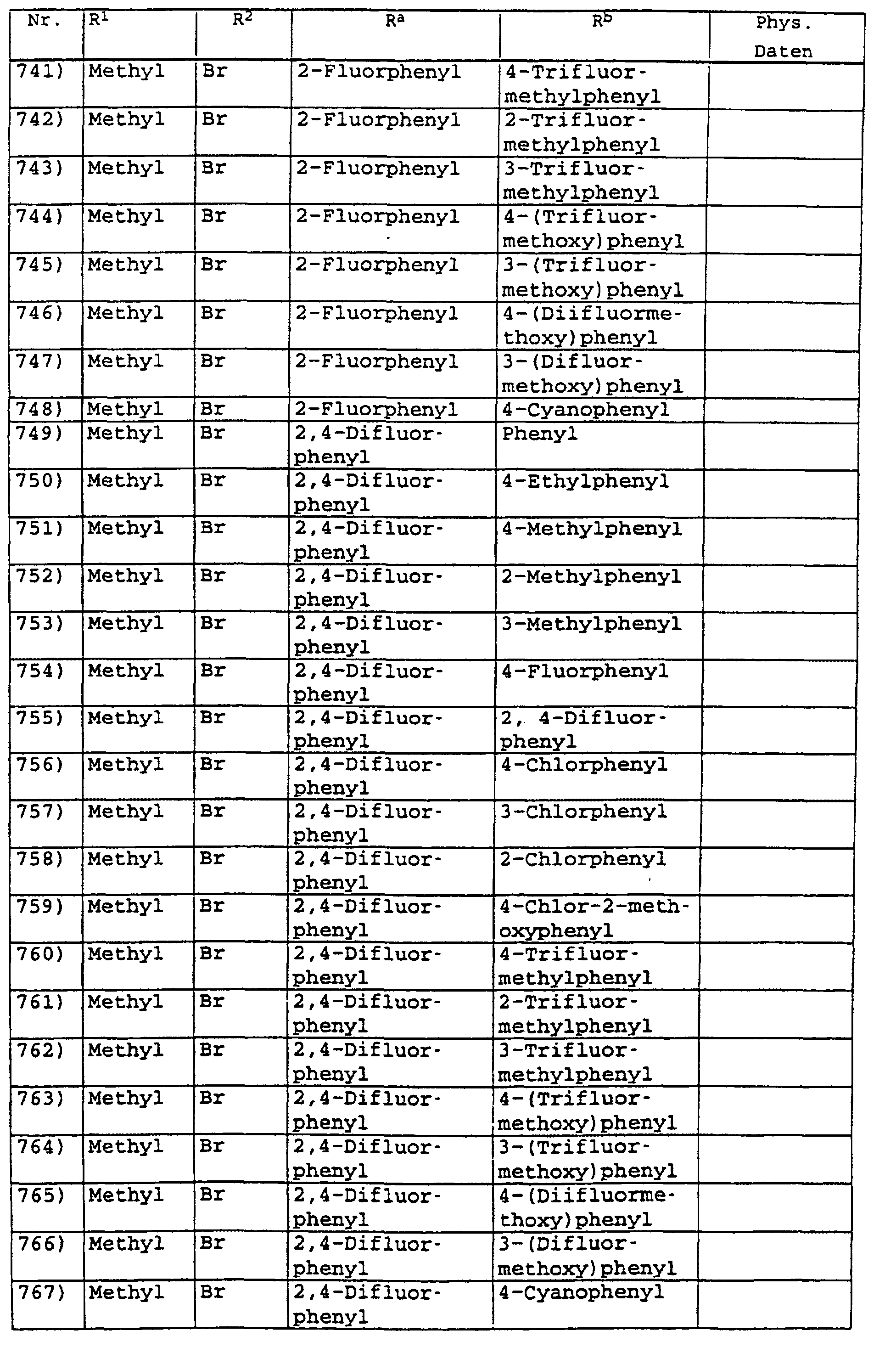

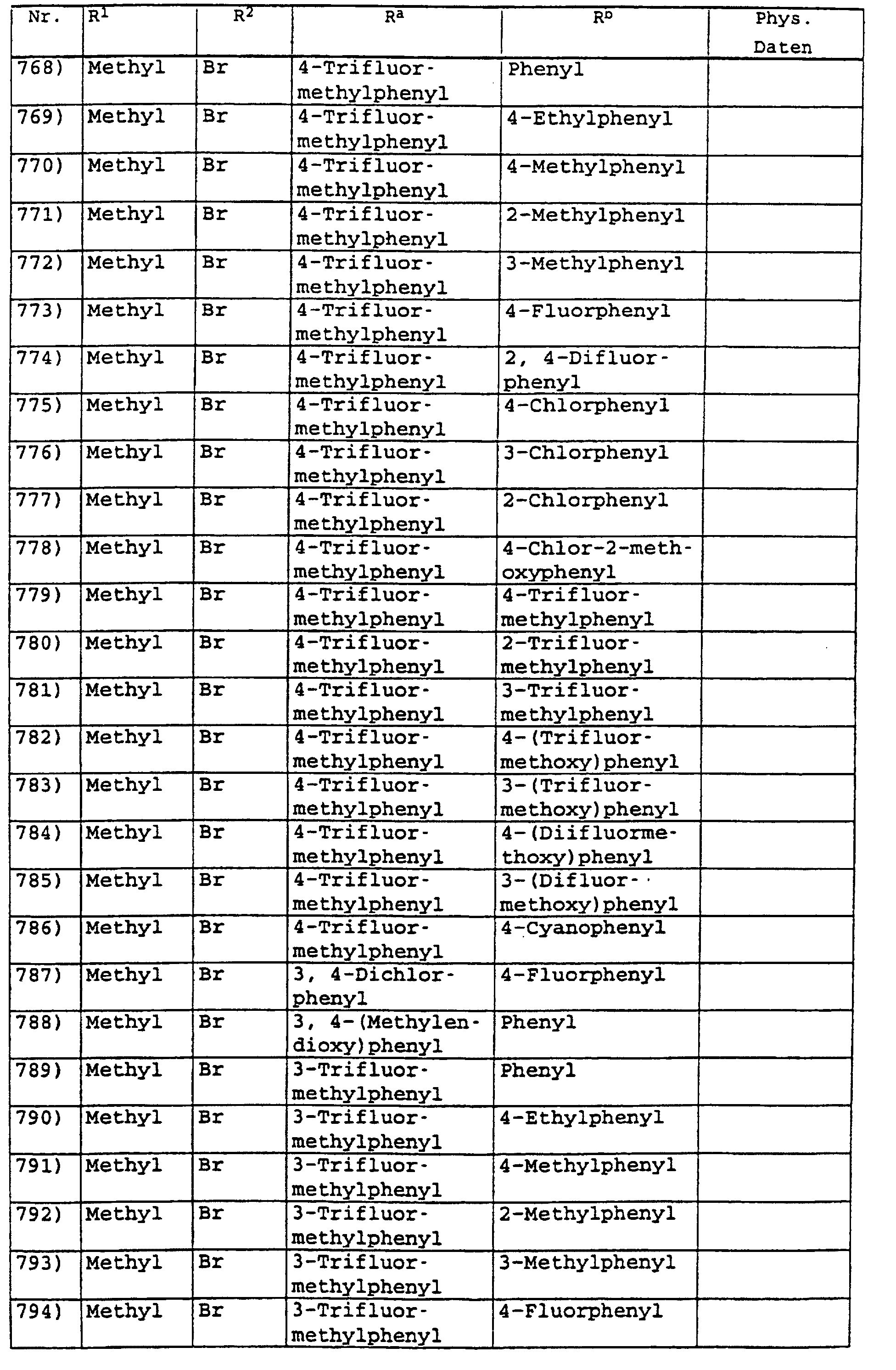

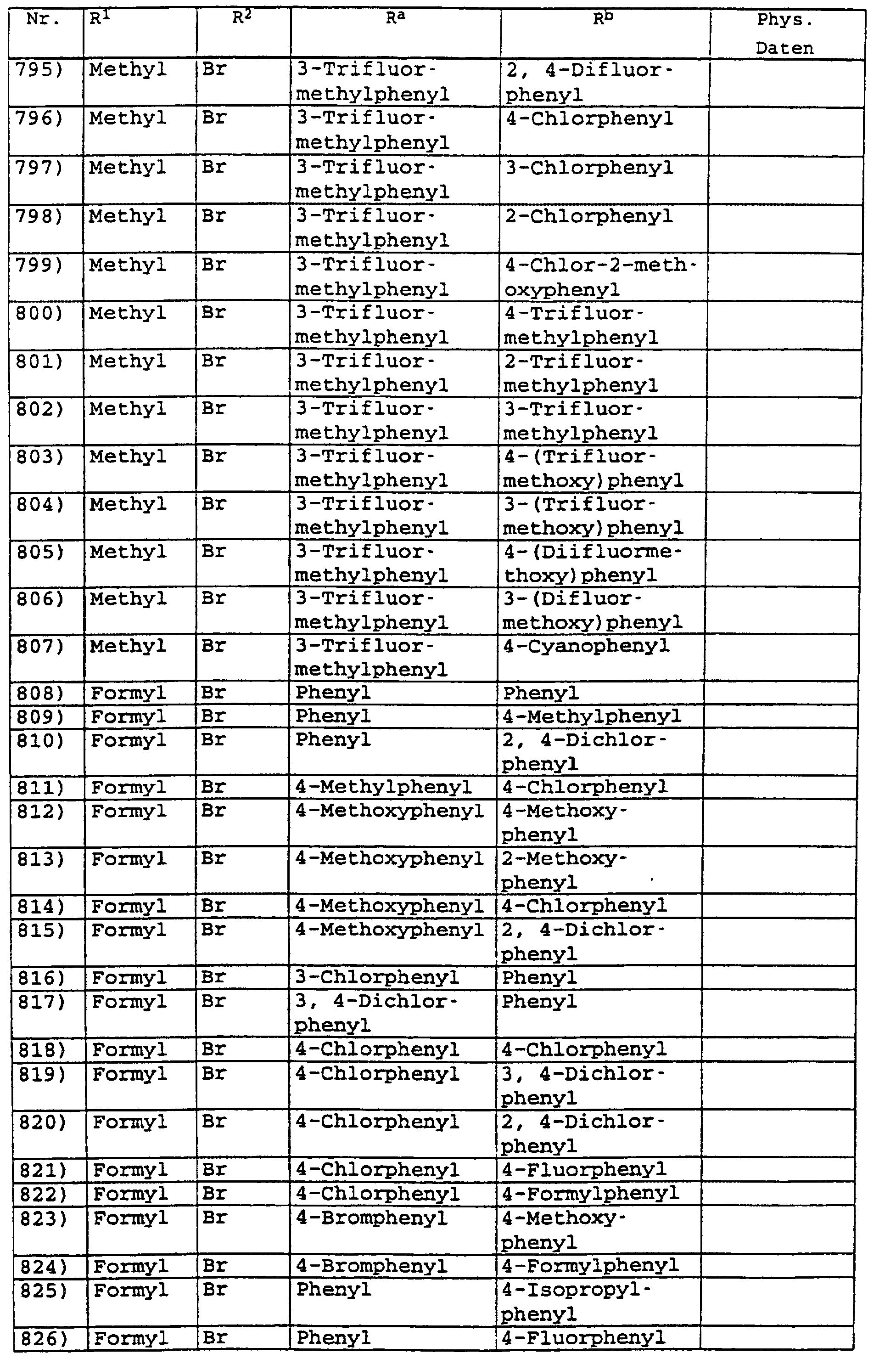

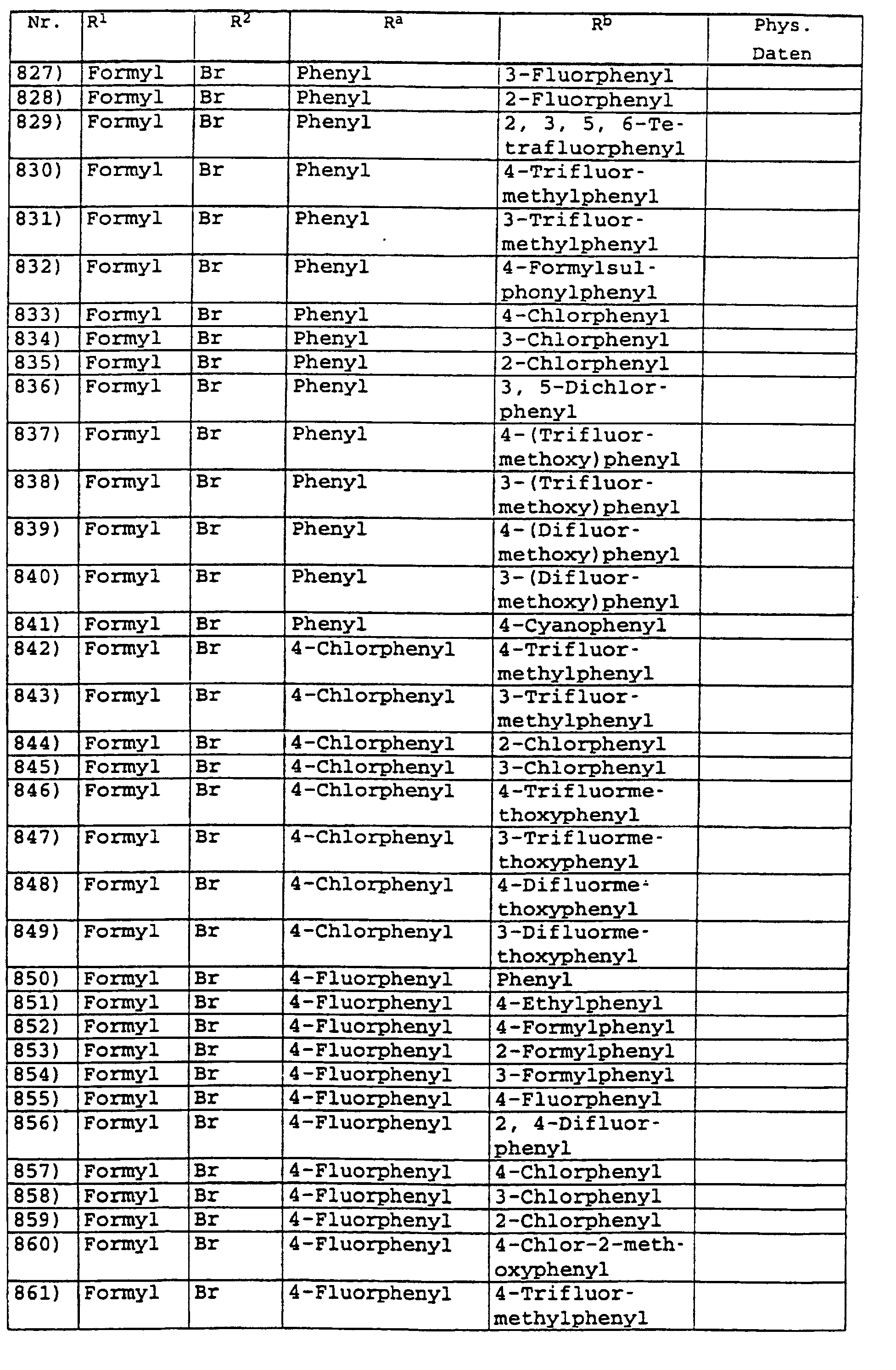

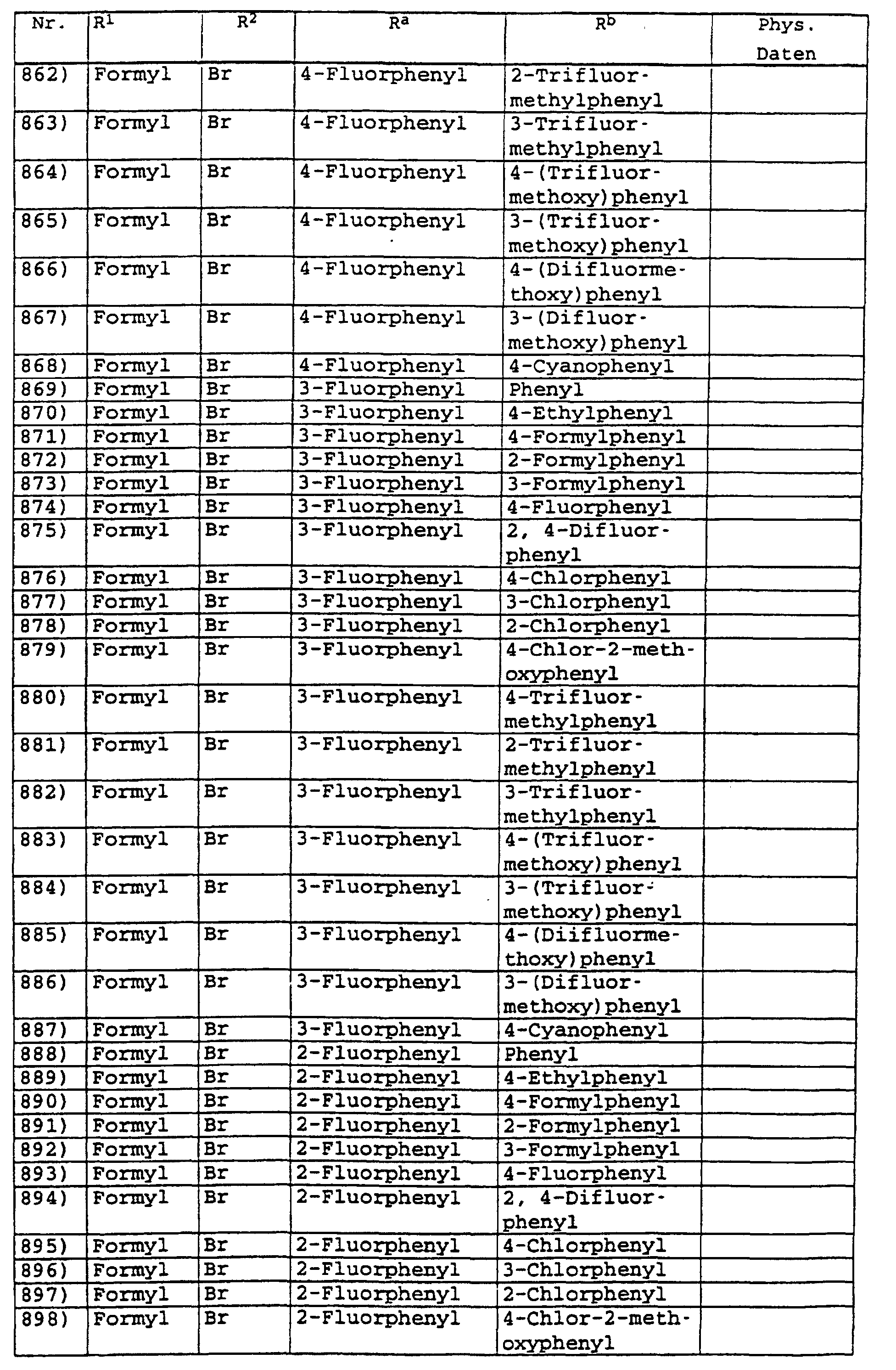

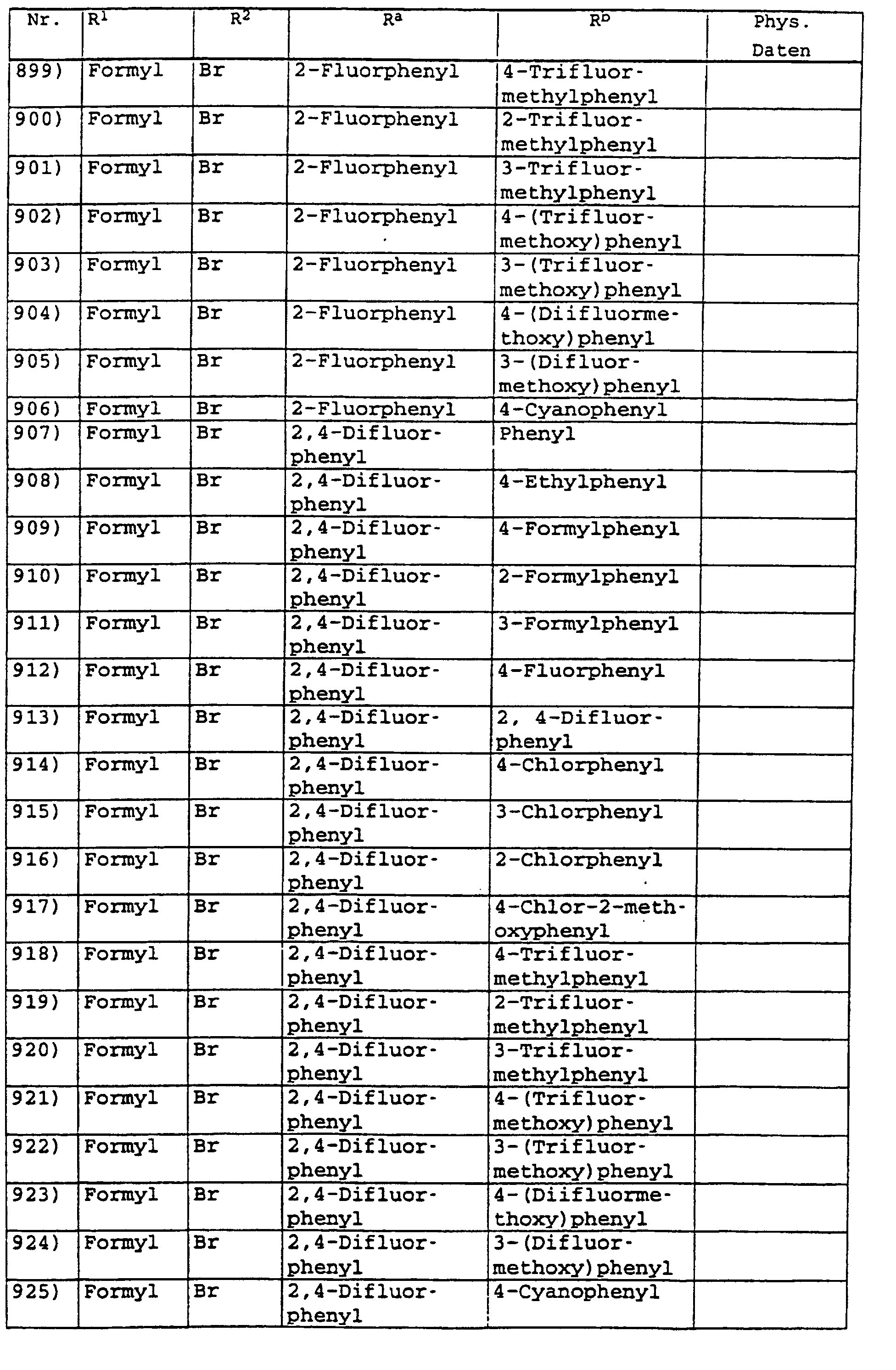

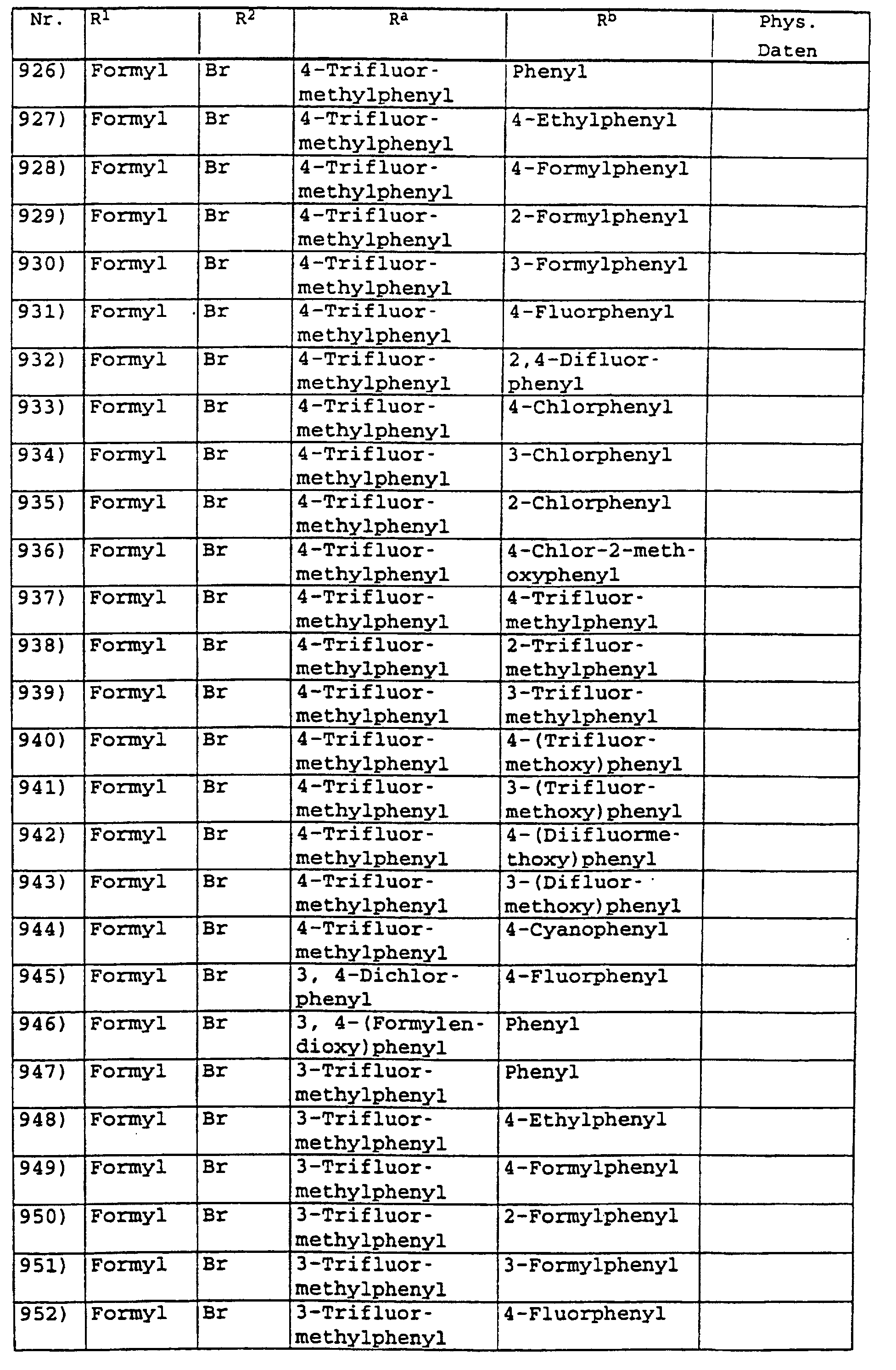

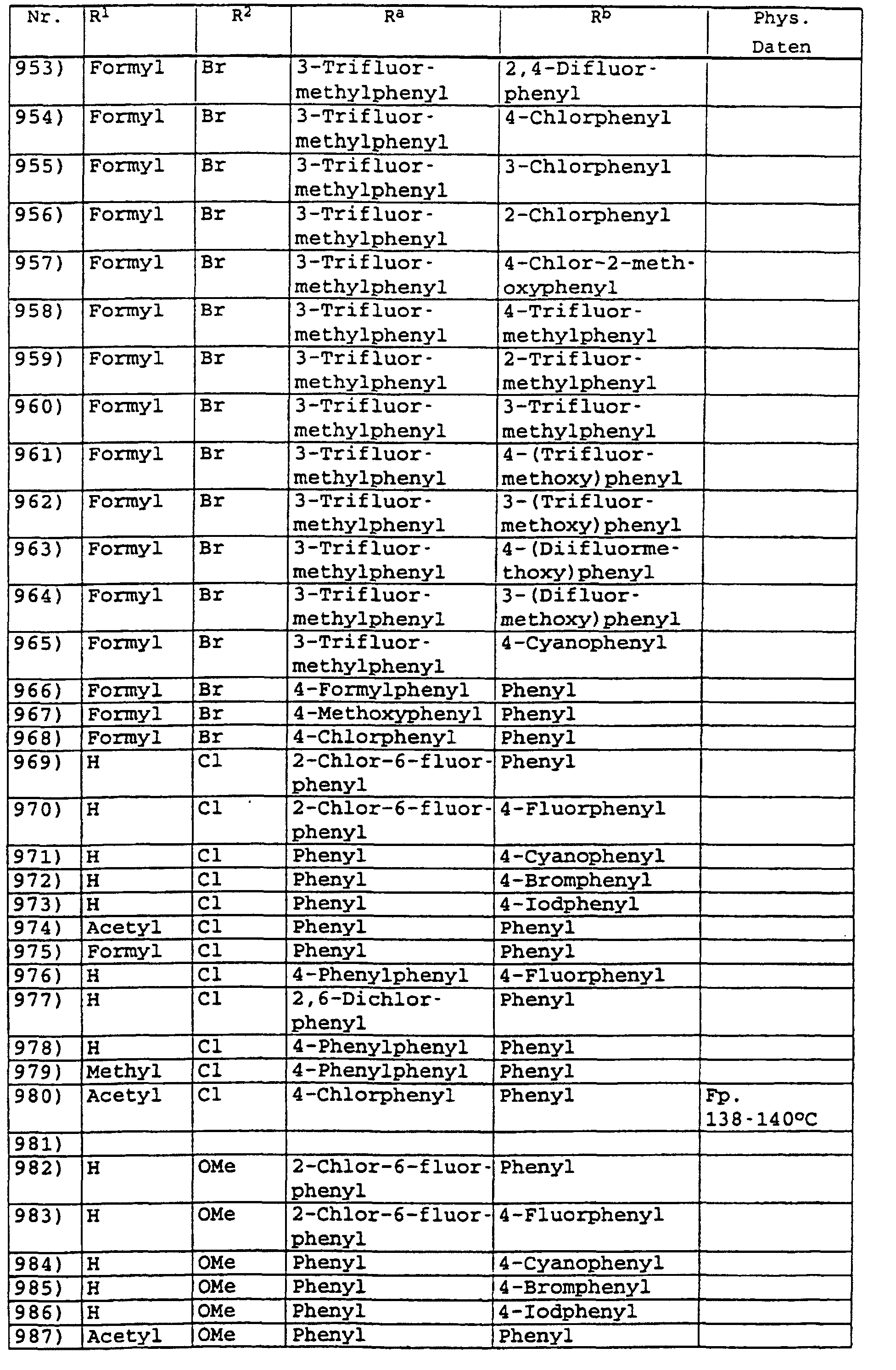

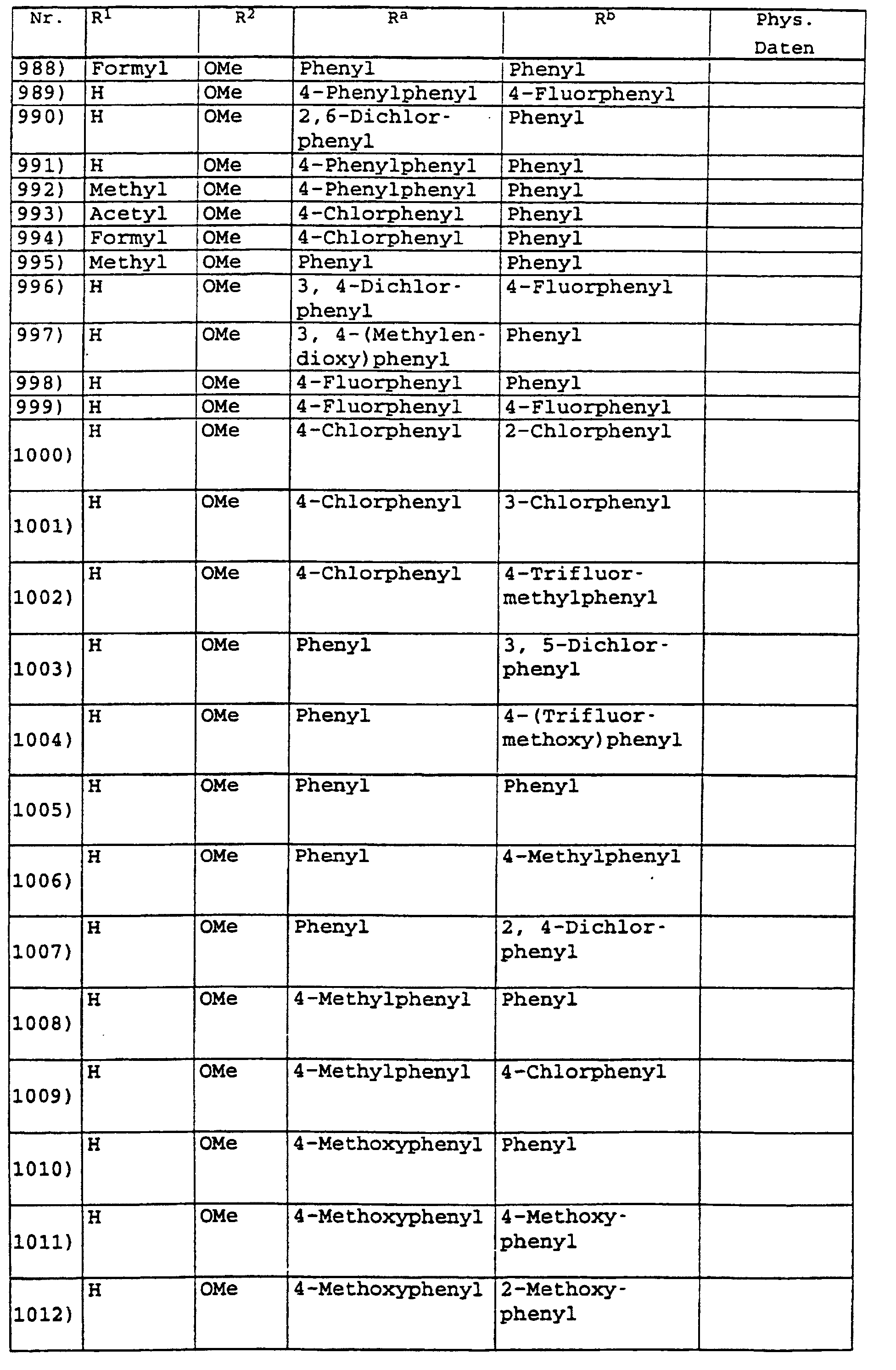

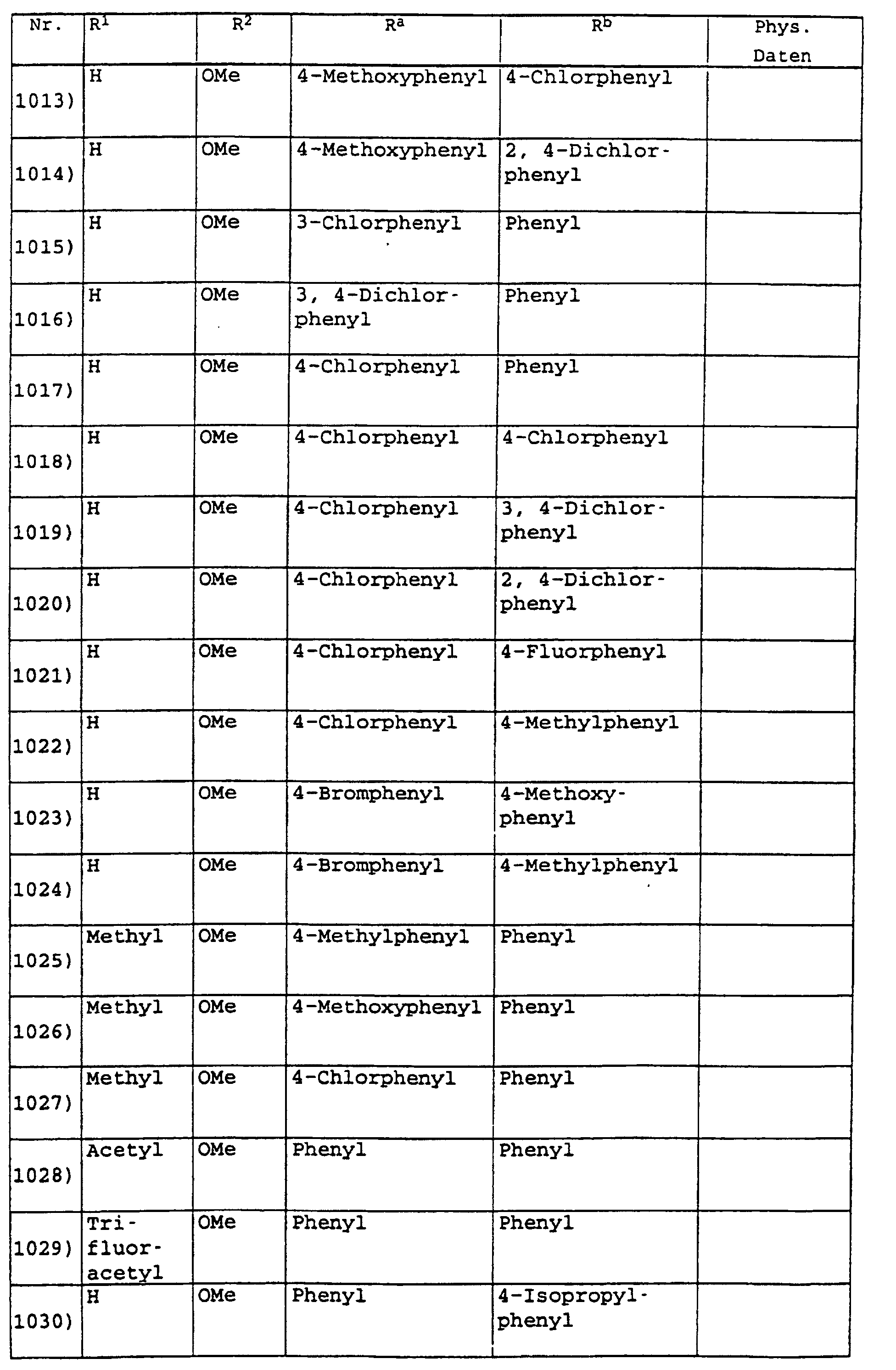

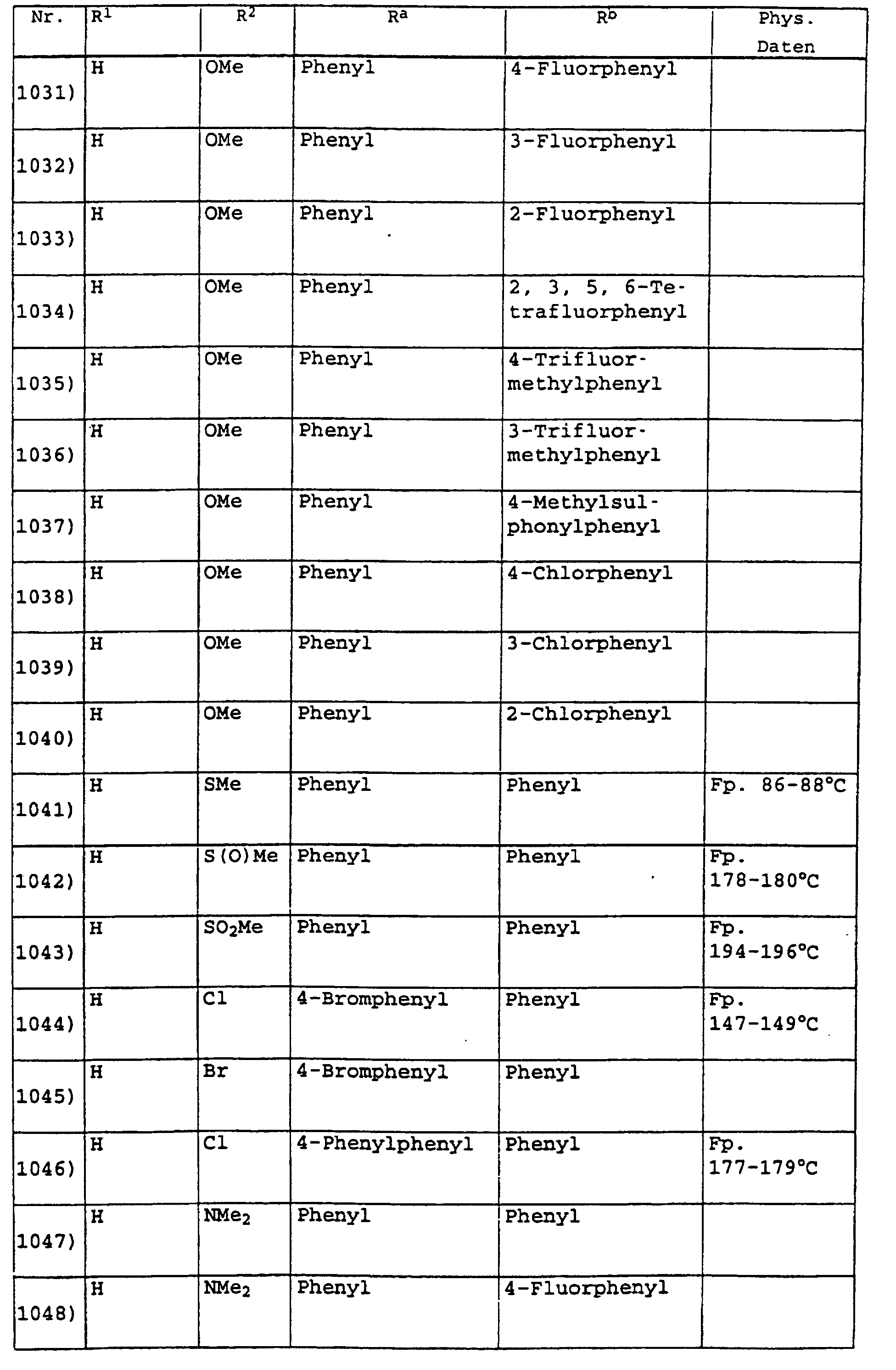

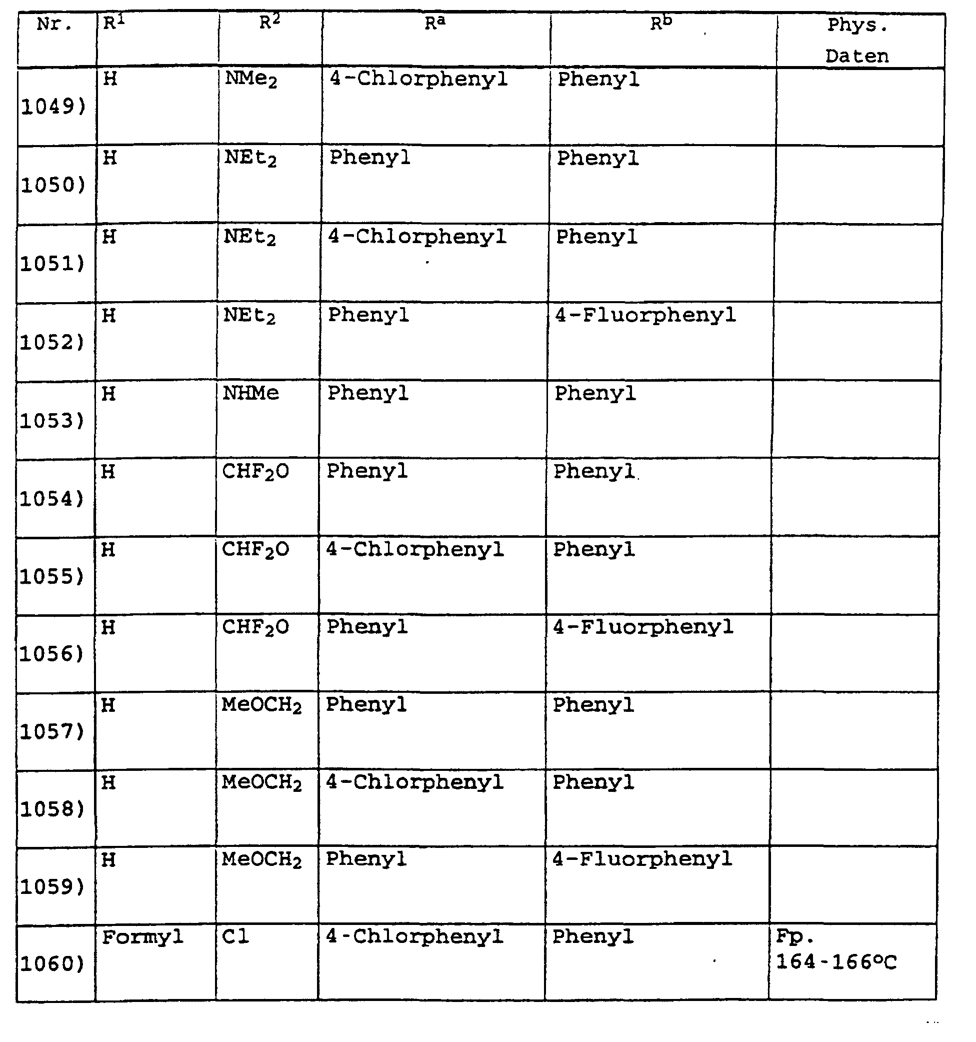

Die Verbindungen der Formel I lassen sich beispielsweise anhand des folgenden Reaktionsschemas herstellen:

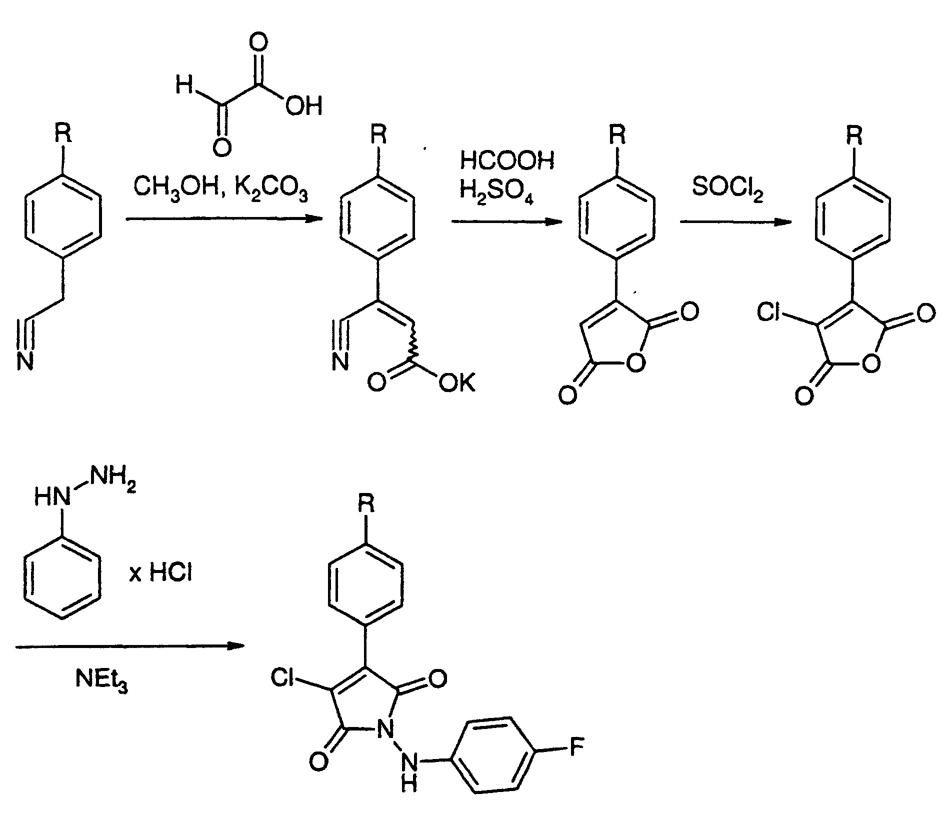

Verbindungen der Formel II sind insbesondere solche, bei denen R¹ ein Wasserstoffatom oder eine C₁-C₄-Alkylgruppe bzw. eine Formylgruppe (-CHO) darstellen. Bevorzugt sind ferner Verbindungen II, bei denen die Reste R², R^{a} und R^{b} unabhängig voneinander folgende Bedeutung haben:
- R²: Halogen, C₁-C₆-Alkoxy, C₁-C₆-Alkylthio, Halogen-C₁-C₆-alkyl, Halogen - C₁- C₆- alkoxy;
- R^{a}: Phenyl, das ein- oder mehrfach, vorzugsweise ein- oder zweifach substituiert sein kann durch Halogen, Halogen-C₁-C₆-alkyl oder durch eine Phenylgruppe, die ihrerseits ebenfalls durch Halogen oder C₁-C₄-Alkyl substituiert sein kann;
- R^{b}: Phenyl, das ein- oder mehrfach, vorzugsweise ein- bis vierfach, substituiert sein kann durch Halogen, C₁-C₆-Alkyl, C₁-C₄-Halogenalkyl, C₁-C₄-Halogenalkoxy.

In diesem Sinne haben die Reste R¹, R^{a} und R^{b} im Fall der Verbindungen II beispielsweise folgende Bedeutung:
- R¹:: Wasserstoff, Methyl, Formyl, Acetyl;
- R²:: Halogen;
- R^{a}:: Phenyl, 4-Chlorphenyl, 4-Fluorphenyl, 3,4-Dichlorphenyl, 2-Chlor-6-fluorphenyl, 4-Phenylphenyl, 2,6-Dichlorphenyl oder 2-Chlorphenyl.
- R^{b}:: 4-Isopropylphenyl, 2,3,5,6-Tetrafluorphenyl, 4-Trifluormethylphenyl, 4-Chlorphenyl, 3-Chlorphenyl, 2-Chlorphenyl, 3,5-Dichlorphenyl, 4-(Trifluormethoxy)phenyl, 4-Trifluormethylphenyl, Phenyl, 4-Fluorphenyl, 4-Cyanophenyl, 4-Bromphenyl, 4-Iodphenyl.

Die Verbindungen I zeichnen sich durch eine hervorragende fungizide Wirkung aus. Dies trifft insbesondere zu für die in der Tabelle 1 genannten Verbindungen Nr. 1, 6, 23, 27, 486, 1041, 1042, 1043, 1044, 1045 und 1046.

Normalerweise werden die Pflanzen mit den Wirkstoffen besprüht oder bestäubt oder die Samen der Pflanzen mit den Wirkstoffen behandelt.

Die Formulierungen (fungiziden Mittel bzw. agrochemischen Zusammensetzungen) werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes iso-Octyl-, Octyl- oder Nonylphenol, Alkylphenol-, Tributylphenylpolyglykolether, Alkylarylpolyetheralkohole, iso-Tridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calciumund Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
II. eine Mischung aus 10 Gew.-Teilen einer erfindungsgemäßen Verbindung I, 70 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 10 Gew.-Teilen einer erfindungsgemäßen Verbindung I, 40 Gew.-Teilen Cyclohexanon, 30 Gew.-Teilen iso-Butanol, 20 Gew.-Teilen des Anlagerungsproduktes von 40 mol Ethylenoxid an 1 mol Ricinusöl;
IV. eine wäßrige Dispersion aus 10 Gew.-Teilen einer erfindungsgemäßen Verbindung I, 25 Gew.-Teilen Cyclohexanol, 55 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen, vorzugsweise einer festen erfindungsgemäßen Verbindung I, 3 Gew.-Teilen des Natriumsalzes der Di-iso-butylnaphthalin-2-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 62 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 50 Gew.-Teilen eines paraffinischen Mineralöls.

Die Wirkstoffe der Formel I zeichnen sich durch eine hervorragende Wirkung gegen ein breites Spektrum von pflanzenpathogenen Pilzen, insbesondere aus der Klasse der Ascomyceten, Deuteromyceten, Phycomyceten und Basidiomyceten, aus. Sie sind z.T. systemisch wirksam und können daher auch als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Wirkstoffe der Formel I können entweder in freier Form oder in Form ihrer landwirtschaftlich einsetzbaren bzw. umweltverträglichen Salze vorliegen. Derartige Salze sind beispielsweise Säureadditionssalze mit anorganischen oder organischen Säuren, z.B. Salzsäure, Schwefelsäure, Essigsäure, u.a. Säuren.

Die Wirkstoffe der Formel I können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,025 und 2, vorzugsweise 0,1 bis 1 kg Wirkstoff pro ha.

Bei der Saatgutbehandlung werden im allgemeinen Wirkstoffmengen von 0,001 bis 50, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit anderen fungiziden Wirkstoffen erhält man dabei in vielen Fällen eine Vergrößerung des fungiziden Wirkungsspektrums. Die Wirkstoffe der Formel I reduzieren insbesondere dann, wenn sie in Kombination mit anderen fungiziden Wirkstoffen eingesetzt werden, die Gefahr einer Resistenzentwicklung im Vergleich zur Anwendung der Einzelwirkstoffe.

Für den Fall, daß die Kulturpflanzen oder das Saatgut mit Kombinationspräparaten von Wirkstoffen der Formel I und anderen fungiziden Wirkstoffen behandelt werden, kann diese Anwendung gleichzeitig oder zeitlich nacheinander erfolgen. Bei gleichzeitiger Anwendung der Wirkstoffe der Formel I mit anderen Fungiziden erfolgt dies zweckmäßigerweise durch Herstellung einer agrochemischen Mischung der beiden Wirkstoffe, wobei diese Mischung zur Behandlung der Kulturpflanzen oder des Saatgutes in üblicher Weise angewandt werden. Bei zeitlich nacheinander erfolgendem Einsatz der Wirkstoffe erfolgt dies zweckmäßigerweise durch Anwendung der Einzelwirkstoffe entweder in kurzem zeitlichem Abstand oder im Abstand von mehreren Tagen oder Wochen. Durch diese kombinierte Anwendung kann insgesamt die Häufigkeit der Behandlung der Pflanzen oder des Saatgutes mit Fungiziden reduziert werden.

Im Sinne der vorliegenden Erfindung versteht man unter dem Begriff "Kombinationspräparate" grundsätzlich alle agrochemischen Zusammensetzungen, die Wirkstoffe der Formel I oder II sowie einen oder mehrere Wirkstoffe, insbesondere mit fungizider Wirkung enthalten, beispielsweise in Form von üblichen agrochemischen Mischungen. Der Begriff "Kombinationspräparate" umfaßt ferner auch solche agrochemischen Zubereitungen, die Wirkstoffe der Formel I enthalten, und die außerdem einen Hinweis enthalten, daß sich diese Wirkstoffe zur kombinierten Anwendung mit anderen Wirkstoffen auf dem Agrarsektor eignen. Ein derartiger Hinweis kann z.B. in Form eines Verpackungsaufdruckes auf der Handelsware oder auf dem Behältnis vorliegen, in dem sich der Wirkstoff der Formel I bzw. das agrochemische Mittel enthaltend einen Wirkstoff der Formel I befindet. Alternativ ist es auch möglich, daß andere agrochemische Erzeugnisse entsprechende Hinweise auf die kombinierte Anwendung mit Verbindungen der Formel I oder II aufweisen. Derartige Erzeugnisse gelten in diesem Sinne ebenfalls als Kombinationspräparate, die zur Anwendung in Kombination mit Wirkstoffen der Formel I bzw. II geeignet sind.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid, N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thion-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, piperazin-1,4-diylbis-(1-(2,2,2-trichlor-ethyl)-formamid, 1-(3,4-Dichloranilino)-1-formylamino-2,2,2-trichlorethan,2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,b-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-ylethyl)-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol, sowie
verschiedene Fungizide, wie Dodecylguanidinacetat, 3-[3-(3,5-Dimethyl-2-oxycyclohexyl)-2-hydroxyethyl]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[(3,5-Dichlorphenyl)-5-methyl-5-methoxymethyl-1,3-oxazolidin-2,4-dion, 3-(3,5-Dichlorphenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-a-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol,
Strobilurine wie Methyl-E-methoximino-[a-(o-tolyloxy)-o-tolyl)acetat, Methyl-E-2-{2-[6-(2-cyanophenoxy)pyridimin-4-yloxy]phenyl}-3-methoxyacrylat, Methyl-E-methoximino-[a-(2,5-dimethyloxy)-o-tolyl)acetamid,
Anilino-Pyrimidine wie N-(4,6-dimethylpyrimidin-2-yl)anilin, N-[4-methyl-6-(1-propinyl)pyrimidin-2-yl)anilin, N-(4-methyl-6-cyclopropyl-pyrimidin-2-yl)anilin,
Phenylpyrrole wie 4-(2,2-difluor-1,3-benzodioxol-4-yl)-pyrrol-3-carbonitril,
Zimtsäureamide wie 3-(4-chlorphenyl)-3-(3,4-dimethoxyphenyl)acrylsäuremorpholid.

Die Erfindung wird anhand der folgenden Ausführungsbeispiele näher erläutert:

### Beispiel 1

### 1-Anilino-3-chlor-4-phenyl-pyrrol-2,5-dion (Tabelle 1, Nr. 1)

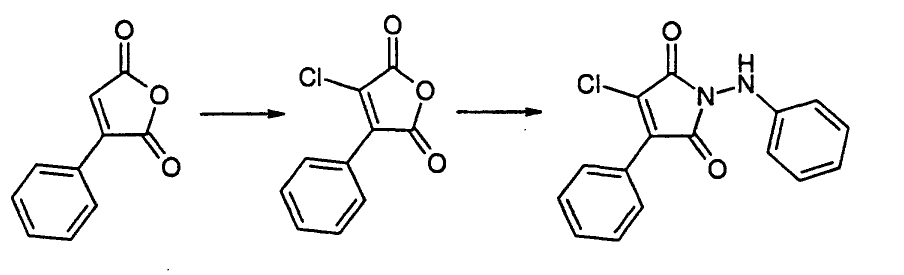

### a) 3-Chlor-4-phenyl-furan-2,5-dion

Unter Eiskühlung wurden 10,0 g (57 mmol) Phenylmaleinsäureanhydrid zu 57 ml Thionylchlorid gegeben und innerhalb von 10 min tropfenweise mit 9,08 g (115 mmol) Pyridin versetzt, wobei die Temperatur bei 10-12°C gehalten wurde. Es wurde 30 min bei 10-12°C nachgerührt, mit einem vorgeheizten Heizbad für 10 min auf 75°C erwärmt, abkühlen lassen und bei 60°C im Vakuum das überschüssige Thionylchlorid abgezogen. Nun wurde mit 120 ml Toluol aufgekocht, filtriert und der Rückstand mit 50 ml heißem Toluol nachgewaschen. Das Filtrat wurde im Vakuum eingeengt, mit Petrolether ausgerührt und getrocknet. Ausbeute: 8,5 g, Schmp. 82-83°C.

### b) 1-Anilino-3-chlor-4-phenyl-pyrrol-2,5-dion

4,14 g (20 mmol) 3-Chlor-4-phenyl-furan-2,5-dion wurden in 50 ml Chloroform vorgelegt und bei Raumtemperatur unter Rühren tropfenweise mit 2,16 g (20 mmol) Phenylhydrazin versetzt und über Nacht bei Raumtemperatur nachgerührt. Es wurde filtriert, dreimal mit Natriumhydrogencarbonat-Lösung und einmal mit Wasser gewaschen, über Natriumsulfat getrocknet und im Vakuum eingeengt. Ausbeute: 5,4 g eines kristallinen Feststoffs, Schmp. 144-146°C.

### Beispiel 2

### 1-Anilino-3-methylthio-4-phenyl-pyrrol-2,5-dion (Tabelle 1, Nr. 1041)

22,3 g (75 mmol) 1-Anilino-3-chlor-4-phenyl-pyrrol-2,5-dion gelöst in 220 ml Dimethylformamid wurden bei Raumtemperatur unter Rühren mit 5,78 g (82 mmol) Natriummethylthiolat versetzt und über Nacht bei Raumtemperatur nachgerührt. Die Mischung wurde in Wasser gegeben, mit Methyl-tert.-butylether extrahiert, der Extrakt mehrfach mit Wasser gewaschen, über Natriumsulfat getrocknet und im Vakuum eingeengt. Der Rückstand wurde zur Reinigung mit Cyclohexan ausgerührt. Ausbeute: 19,4 g Feststoff vom Schmp. 86-88°C.

### Beispiel 3

### 3-Chlor-1-((4-fluorphenyl)amino)-4-phenyl-pyrrol-2,5-dion (Tabelle 1, Nr. 27)

3,13 g (15 mmol) 3-Chlor-4-phenyl-furan-2,5-dion und 2,44 g (15 mmol) 4-Fluorphenylhydrazinhydrochlorid wurden in 50 ml Methylenchlorid vorgelegt und bei Raumtemperatur unter Rühren tropfenweise mit 1,52 g (15 mmol) Triethylamin versetzt und über Nacht bei Raumtemperatur nachgerührt. Es wurde weitere 50 ml Methylenchlorid zugegeben, dreimal mit je 80 ml Wasser gewaschen, über Natriumsulfat getrocknet und im Vakuum eingeengt. Ausbeute: 3,9 g eines kristallinen Feststoffs, Schmp. 115-117°C.

### Beispiel 4

### 3-Chlor-4-(4-chlorphenyl)-1-(N-methyl-N-phenylamino)-pyrrol-2,5-dion (Tabelle 1, Nr. 23)

### a) Kalium-3-(4-chlorphenyl)-3-cyanoacrylat

Zu 91,2 g (0,6 Mol) (4-Chlorphenyl)acetonitril und 210 g (1,5 Mol) Kaliumcarbonat in 1,2 1 Methanol wurden 133 g (0,9 Mol) 50%ige wässrige Glyoxylsäure unter Rühren so zugetropft, daß die Reaktionstemperatur auf 35°C anstieg. Es wurde 6 h bei Raumtemperatur nachgerührt, filtriert, der Rückstand mit Methylenchlorid gewaschen und im Vakuum getrocknet. Das Rohprodukt wurde 1,5 h mit 3 1 Wasser bei Raumtemperatur verrührt, der Rückstand abfiltriert, mit Wasser gewaschen und getrocknet. Ausbeute 138 g Feststoff vom Schmp. 241-242°C.

### b) 3-(4-Chlorphenyl)-furan-2,5-dion

Zu 138 g (0,57 Mol) Kalium-3-(4-chlorphenyl)-3-cyanoacrylat in 1,2 l 88%iger Ameisensäure wurden unter Rühren 80 ml konzentrierte Schwefelsäure zugetropft. Dabei stieg die Temperatur auf 50°C an. Es wurde 3 h unter Rückfluß gekocht, nach dem Abkühlen auf 10 l Eiswasser gegossen, 1 h nachgerührt und das Produkt abfiltriert, mit Wasser gewaschen und im Vakuum getrocknet. Ausbeute 86,0 g vom Schmp. 144-145°C.

### c) 3-Chlor-4-(4-chlorphenyl)-furan-2,5-dion

Unter Eiskühlung wurden 15,0 g (72 mmol) 3-(4-Chlorphenyl)-furan-2,5-dion zu 72 ml Thionylchlorid gegeben und innerhalb von 10 min tropfenweise mit 11,5 g (145 mmol) Pyridin versetzt, wobei die Temperatur bei 10-12°C gehalten wurde. Es wurde 30 min bei 10-12°C nachgerührt, mit einem vorgeheizten Heizbad für 10 min auf 75°C erwärmt, abkühlen lassen und bei 60°C im Vakuum das überschüssige Thionylchlorid entfernt. Nun wurde mit 200 ml Toluol aufgekocht, filtriert und der Rückstand nochmals mit 100 ml aufgekocht und heiß abfiltriert. Das Filtrat wurde im Vakuum eingeengt, der Rückstand mit 100 ml Petrolether aufgekocht, abgekühlt, abfiltriert, nochmals mit Petrolether gewaschen und im Vakuum getrocknet. Ausbeute: 16,2 g, Schmp. 110-112°C.

### d) 3-Chlor-4-(4-chlorphenyl)-1-(N-methyl-N-phenylamino)-pyrrol-2,5-dion

3,65 g (15 mmol) 3-Chlor-4-(4-chlorphenyl)-furan-2,5-dion wurden in 75 ml Chloroform vorgelegt und bei Raumtemperatur unter Rühren tropfenweise mit 1,83 g (15 mmol) N-Methyl-N-phenylhydrazin gelöst in 15 ml Chloroform versetzt und über Nacht bei Raumtemperatur nachgerührt. Nach dem Einengen im Vakuum wurde in 200 ml Methylenchlorid aufgenommen, dreimal mit je 150 ml Wasser gewaschen, über Natriumsulfat getrocknet und im Vakuum eingeengt. Ausbeute: 4,6 g eines kristallinen Feststoffs, Schmp. 155-157°C.

### Beispiel 5

### 1-Anilino-3-methylsulfinyl-4-phenyl-pyrrol-2,5-dion (Tabelle 1, Nr. 1042) und 1-Anilino-3-methylsulfonyl-4-phenyl-pyrrol-2,5-dion (Tabelle 1, Nr. 1043)

2,0 g (6,5 mmol) 1-Anilino-3-methylthio-4-phenyl-pyrrol-2,5-dion und 69 mg (0,2 mmol) Natriumwolframat-Dihydrat in 20 ml Essigsäure wurden bei 40°C unter Rühren tropfenweise mit 1,5 g (13 mmol) 30 %igem Wasserstoffperoxid versetzt und 3 h bei dieser Temperatur gehalten. Dann wurden nochmals 0,3 g (2,6 mmol) 30 %iges Wasserstoffperoxid zugegeben und 4 h bei 40°C sowie über Nacht bei Raumtemperatur gerührt. Dann wurde auf 80 ml Wasser gegossen, das Rohprodukt abfiltriert, mit etwas Wasser gewaschen und im Vakuum getrocknet. Durch Chromatographie mit Essigsäureethylester/Cyclohexan an Kieselgel wurden 0,45 g 1-Anilino-3-methylsulfinyl-4-phenyl-pyrrol-2,5-dion vom Schmp. 178-180°C und 0,65 g 1-Anilino-3-methylsulfonyl-4-phenyl-pyrrol-2,5-dion vom Schmp. 194-196°C isoliert.

### Beispiel 6

### 1-Anilino-3-brom-4-phenyl-pyrrol-2,5-dion (Tabelle 1, Nr. 486)

### a) 3-Brom-4-phenyl-furan-2,5-dion

Bei 10°C wurden zu 16,8 g (97 mmol) Phenylmaleinsäureanhydrid in 200 ml Toluol zunächst 40,2 g (193 mmol) Thionylbromid und dann 15,3 g (193 mmol) Pyridin unter Rühren zugetropft. Es wurde 30 min bei 10°C nachgerührt, mit einem vorgeheizten Heizbad für 30 min auf 75°C erwärmt, abkühlen lassen und bei 65°C im Vakuum das überschüssige Thionylbromid entfernt. Nun wurde mit 150 ml Toluol verrührt, filtriert und der Rückstand zweimal mit je 200 ml Toluol nachgewaschen und das Filtrat im Vakuum eingeengt. Das Rohprodukt (9,3 g) enthielt noch 25% unumgesetztes Ausgangsmaterial und wurde ohne weitere Reinigung eingesetzt.

### b) 1-Anilino-3-brom-4-phenyl-pyrrol-2,5-dion

4,3 g (17 mmol) 3-Brom-4-phenyl-furan-2,5-dion wurden in 40 ml Chloroform vorgelegt, bei Raumtemperatur unter Rühren tropfenweise mit 1,84 g (17 mmol) Phenylhydrazin in 15 ml Chloroform versetzt und über Nacht bei Raumtemperatur nachgerührt. Es wurde filtriert, im Vakuum eingeengt und durch Chromatographie an Kieselgel mit Essigsäureethylöester/Cyclohexan gereinigt. Ausbeute: 1,9 g, Schmp. 146-147°C.

### Beispiel 7

### Wirksamkeit gegen Phytophthora infestans an Tomaten

Blätter von Topfpflanzen der Sorte "Große Fleischtomate" wurden mit einer wäßrigen Suspension, die aus einer Stammlösung aus 10 % Wirkstoff, 63 % Cyclohexanon und 27 % Emulgiermittel angesetzt wurde, bis zur Tropfnässe besprüht. Am folgenden Tag wurden die Blätter mit einer wäßrigen Zoosporenaufschwemmung von *Phytophthora infestans* infiziert. Anschließend wurden die Pflanzen in einer wasserdampfgesättigten Kammer bei Temperaturen zwischen 16 und 18°C aufgestellt. Nach 6 Tagen hatte sich die Krautfäule auf den unbehandelten, jedoch infizierten Kontrollpflanzen so stark entwickelt, daß der Befall visuell in % ermittelt werden konnte.

| Wirkstoff | %-Befall der Blätter nach Applikation von 250 ppm-haltiger wäßriger Wirkstoffaufbereitung |
|---|---|
| Verbindung (I) | |
| Unbehandelt | 90 |

Aus den Untersuchungen geht hervor, daß bei den behandelten Pflanzen eine durch Schadpilze verursachte deutlich geringere Schädigung zu beobachten ist als im Vergleich zu den unbehandelten Pflanzen. Die erfindungsgemäßen Wirkstoffe weisen demzufolge eine gute fungizide Wirkung auf. Sie besitzen insbesondere einen protektiven Effekt gegen Schadpilze.

### Beispiel 8

### Wirksamkeit gegen Plasmopara viticola

Blätter von Topfreben der Sorte "Müller-Thurgau" wurden mit wäßriger Wirkstoffaufbereitung, die mit einer Stammlösung aus 10 % Wirkstoff, 63 % Cyclohexanon und 27 % Emulgiermittel angesetzt wurde, bis zur Tropfnässe besprüht. Um die Dauerwirkung der Substanzen beurteilen zu können, wurden die Pflanzen nach dem Antrocknen des Spritzbelages für 7 Tage im Gewächshaus aufgestellt. Erst dann wurden die Blätter mit einer wäßrigen Zoosporenaufschwemmung von *Plasmopara viticola* inokuliert. Danach wurden die Reben zunächst für 48 Stunden in einer wasserdampfgesättigten Kammer bei 24° C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

| Wirkstoff | %-Befall der Blätter nach Applikation von 250 ppm-haltiger wäßriger Wirkstoffaufbereitung |
|---|---|
| Verbindung (I) Unbehandelt | 85 |

## Patentansprüche

1. Agrochemische Zusammensetzung mit fungizider Wirkung enthaltend als Wirkstoffe Verbindungen der Formel I 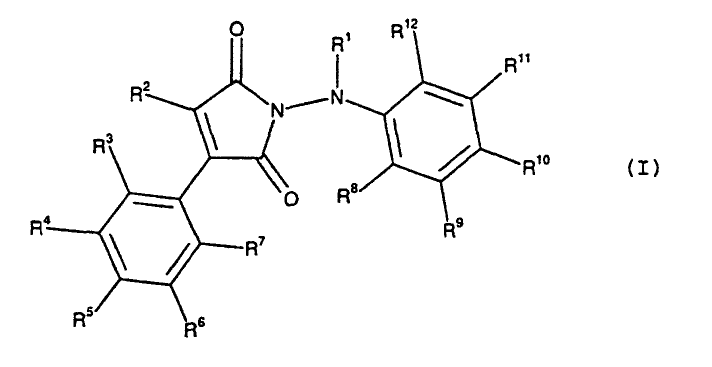 wobei die Reste folgende Bedeutungen haben:
R¹ Wasserstoff, C₁-C₆-Alkyl, C₁-C₆-Alkylcarbonyl, Formyl oder C₁-C₆-Halogenalkylcarbonyl;
R² Halogen, C₁-C₆-Alkylthio, C₁-C₆-Alkoxy, C₃-C₆-Cycloalkyl-C₁-C₆-alkoxy, C₁-C₆-Alkoxy-C₁-C₆-alkyl, Halogen-C₁-C₆-alkoxy, C₁-C₆-Alkylsulfonyl, C₁-C₆-Alkylsulfinyl, Halogen-C₁-C₆-alkylsulfonyl, Cyano oder ein Rest NR¹³R¹⁴;
R³ - R¹² Wasserstoff, Halogen, C₁-C₈-Cycloalkyl, C₁-C₆-Alkyl, Halogen-C₁-C₆-alkyl, C₁-C₆-Alkoxy, Halogen-C₁-C₆-alkoxy, C₁-C₆-Alkylsulfonyl, Halogen-C₁-C₆-alkylsulfonyl, Formyl, C₁-C₆-Alkylcarbonyl, Cyano, C₁-C₆-Alkylthio oder Phenyl, welches ggf. durch Halogenatome, C₁-C₆-Alkyl- oder Halogen-C₁-C₆-alkyl-Gruppen substituiert sein kann,
R¹³ Wasserstoff, C₁-C₆-Alkyl,
R¹⁴ C₁-C₆-Alkyl, C₁-C₈-Cycloalkyl oder gemeinsam mit R¹³ und dem Stickstoffatom, an das sie gebunden sind, einen gesättigten oder ungesätttigten heterocylischen fünf- oder sechsgliedrigen Ring bedeuten, der ein oder zwei Heteroatome ausgewählt aus der Gruppe Stickstoffatom oder Sauerstoffatom enthält,
sowie deren landwirtschaftlich einsetzbaren Salze.

2. Zusammensetzung nach Anspruch 1, wobei R¹ Wasserstoff, C₁-C₆-Alkyl, C₁-C₆-Alkylcarbonyl oder Formyl bedeutet.

3. Zusammensetzung nach Anspruch 2, wobei R¹ Wasserstoff, Methyl, Formyl oder Acetyl bedeutet.

4. Zusammenfassung nach einem der Ansprüche 1-3, wobei R² Halogen, C₁-C₆-Alkylthio, C₁-C₆-Alkylsuflonyl, C₁-C₆-Alkylsulfinyl oder Halogen-C₁-C₆-alkoxy bedeutet.

5. Zusammensetzung nach Anspruch 4, wobei R² Chlor, Brom, Methylthio, Methylsulfonyl, Methylsulfinyl oder Difluormethoxy bedeutet.

6. Zusammensetzung nach einem der Ansprüche 1-5, wobei ein oder mehrere der Reste R³-R¹² die folgenden Bedeutungen haben: Fluor, Chlor, Methyl, Ethyl, Propyl, Butyl, Trifluormethyl, Difluormethyl, Trifluormethoxy, Difluormethoxy, Methoxy, Methylthio, Cyano, und mindestens drei der Reste R³-R¹² Wasserstoff bedeuten.

7. Zusammensetzung nach Anspruch 6, wobei vier bis neun der Reste R³-R¹² Wasserstoff bedeuten.

8. Zusammensetzung nach einem der Ansprüche 1-7, wobei zwei bis fünf der Reste R⁸-R¹² Wasserstoff bedeuten.

9. Zusammensetzung nach Anspruch 8, wobei drei oder vier der Reste R⁸-R¹² Wasserstoff bedeuten.

10. Zusammensetzung nach einem der Ansprüche 1- 9, wobei zwei bis fünf der Reste R³-R⁷ Wasserstoff bedeuten.

11. Zusammensetzung nach Anspruch 10, wobei drei oder vier der Reste R³-R⁷ Wasserstoff bedeuten.

12. Zusammensetzung nach einem der Ansprüche 1 - 11, wobei mindestens zwei der Reste R⁸-R¹² sowie mindestens zwei der Reste R³-R⁷ Wasserstoff bedeuten.

13. Zusammensetzung nach einem der Ansprüche 1 - 12, wobei einer, zwei oder drei der Reste R³-R¹² Halogen, C₁-C₆-Alkyl, C₁-C₆-Halogenalkyl, C₁-C₆-Alkoxy oder C₁-C₆-Halogenalkoxy und die anderen Reste R³-R¹² Wasserstoff bedeuten.

14. Zusammensetzung nach Anspruch 13, wobei die Reste R³-R¹² ausgewählt sind aus Fluor, Chlor, Brom, Jod, Methyl, Ethyl, Propyl, Butyl, Trifluormethyl, Trifluormethoxy oder Difluormethoxy.

15. Zusammensetzung nach einem der Ansprüche 1-14 ausgewählt aus der Gruppe der folgenden Verbindungen:
1-Anilino-3-chlor-4-phenyl-pyrrol-2,5-dion,
1-Anilino-3-methylthio-4-phenyl-pyrrol-2,5-dion,
3-Chlor-4-(4-chlorphenyl)-1-(N-methyl-N-phenylamino)-pyrrol-2,5-dion,
1-Anilino-3-methylsulfinyl-4-phenyl-pyrrol-2,5-dion,
1-Anilino-3-methylsulfonyl-4-phenyl-pyrrol-2,5-dion,
1-Anilino-3-brom-4-phenyl-pyrrol-2,5-dion.

16. Verbindungen der Formel II 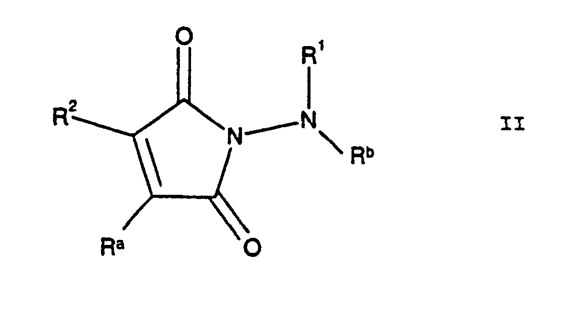 wobei die Reste R¹, R², R^{a} und R^{b} folgende Bedeutungen haben:
R¹ Wasserstoff, C₁-C₄-Alkyl, Formyl;
R² Halogen, C₁-C₆-Alkoxy, C₁-C₆-Alkylthio, C₁-C₆-Alkylsulfonyl, C₁-C₆-Alkylsulfinyl oder Halogen-C₁-C₆-alkoxy bedeutet;
R^{a} Phenyl, das ein- oder mehrfach, vorzugsweise ein- oder zweifach substituiert sein kann durch Halogen, oder durch eine Phenylgruppe, die ihrerseits ebenfalls durch Halogen oder C₁-C₄-Alkyl substituiert sein kann;
R^{b} Phenyl, das ein- oder mehrfach, vorzugsweise ein- bis vierfach, substituiert sein kann durch Halogen, C₁-C₆-Alkyl, C₁-C₄-Halogenalkyl, C₁-C₄-Halogenalkoxy.

17. Verwendung von fungiziden Mitteln nach einem der Ansprüche 1-15 oder von Verbindungen der Formel II nach Anspruch 16 zur Bekämpfung von Schadpilzen in der Landwirtschaft.

18. Verfahren zur Bekämpfung von Schadpilzen, **dadurch gekennzeichnet, daß** man die Schadpilze, deren Lebensraum oder die von ihnen freizuhaltenden Pflanzen, Flächen, Materialien oder Räume mit einer fungizid wirksamen Menge eines Mittels gemäß einem der Ansprüche 1-15 oder von Verbindungen der Formel II nach Anspruch 16 behandelt.

19. Agrochemische Kombinationspräparate enthaltend als Wirkstoffe Verbindungen der Formel I gemäß den Ansprüchen 1-15 oder Verbindungen der Formel II nach Anspruch 16, sowie mindestens einen weiteren fungiziden Wirkstoff.

20. Verwendung von fungiziden Wirkstoffen in Kombination mit mindestens einem wirkstoff der Formel I gemäß einem der Ansprüche 1-15 oder von Verbindungen der Formel II nach Anspruch 16 zur Bekämpfung von Schadpilzen in der Landwirtschaft.

## Claims

1. An agrochemical composition having fungicidal action, comprising as active compounds compounds of the formula I 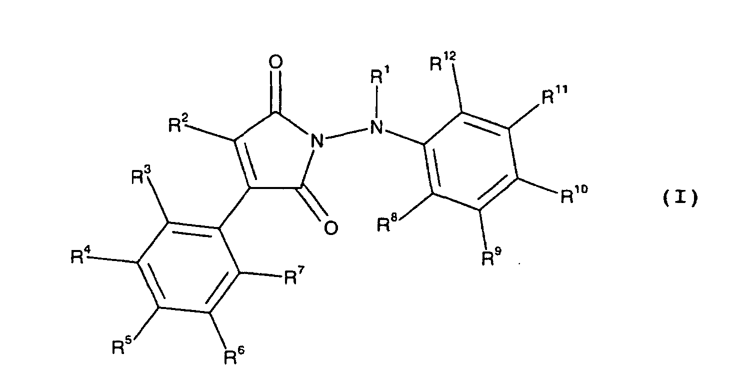 where:
R¹ is hydrogen, C₁-C₆-alkyl, C₁-C₆-alkylcarbonyl, formyl or C₁-C₆-haloalkylcarbonyl;
R² is halogen, C₁-C₆-alkylthio, C₁-C₆-alkoxy, C₃-C₆-cycloalkyl-C₁-C₆-alkoxy, C₁-C₆-alkoxy-C₁-C₆-alkyl, halo-C₁-C₆-alkoxy, C₁-C₆-alkylsulfonyl, C₁-C₆-alkylsulfinyl, halo-C₁-C₆-alkylsulfonyl, cyano or a radical NR¹³R¹⁴;
R³ - R¹² are hydrogen, halogen, C₁-C₈-cycloalkyl, C₁-C₆-alkyl, halo-C₁-C₆-alkyl, C₁-C₆-alkoxy, halo-C₁-C₆-alkoxy, C₁-C₆-alkylsulfonyl, halo-C₁-C₆-alkylsulfonyl, formyl, C₁-C₆-alkylcarbonyl, cyano, C₁-C₆-alkylthio or phenyl, which may be unsubstituted or substituted by halogen, C₁-C₆-alkyl or halo-C₁-C₆-alkyl,
R¹³ is hydrogen, C₁-C₆-alkyl,
R¹⁴ is C₁-C₆-alkyl, C₁-C₈-cycloalkyl or, together with R¹³ and the nitrogen atom to which they are attached, a saturated or unsaturated heterocyclic five- or six-membered ring which contains one or two heteroatoms selected from the group consisting of nitrogen and oxygen,
and its agriculturally useful salts.

2. A composition as claimed in claim 1, wherein R¹ is hydrogen, C₁-C₆-alkyl, C₁-C₆-alkylcarbonyl or formyl.

3. A composition as claimed in claim 2, wherein R¹ is hydrogen, methyl, formyl or acetyl.

4. A composition as claimed in any of claims 1-3, wherein R² is halogen, C₁-C₆-alkylthio, C₁-C₆-alkylsulfonyl, C₁-C₆-alkylsulfinyl or halo-C₁-C₆-alkoxy.

5. A composition as claimed in claim 4, wherein R² is chlorine, bromine, methylthio, methylsulfonyl, methylsulfinyl or difluoromethoxy.

6. A composition as claimed in any of claims 1-5, wherein one or more of the radicals R³-R¹² have the following meanings: fluorine, chlorine, methyl, ethyl, propyl, butyl, trifluoromethyl, difluoromethyl, trifluoromethoxy, difluoromethoxy, methoxy, methylthio, cyano, and at least three of the radicals R³-R¹² are hydrogen.

7. A composition as claimed in claim 6, wherein four to nine of the radicals R³-R¹² are hydrogen.

8. A composition as claimed in any of claims 1-7, wherein two to five of the radicals R⁸-R¹² are hydrogen.

9. A composition as claimed in claim 8, wherein three or four of the radicals R⁸-R¹² are hydrogen.

10. A composition as claimed in any of claims 1-9, wherein two to five of the radicals R³-R⁷ are hydrogen.

11. A composition as claimed in claim 10, wherein three or four of the radicals R³-R⁷ are hydrogen.

12. A composition as claimed in any of claims 1 - 11, wherein at least two of the radicals R⁸-R¹² and at least two of the radicals R³-R⁷ are hydrogen.

13. A composition as claimed in any of claims 1 - 12, wherein one, two or three of the radicals R³-R¹² are halogen, C₁-C₆-alkyl, C₁-C₆-haloalkyl, C₁-C₆-alkoxy or C₁-C₆-haloalkoxy and the other radicals R³-R¹² are hydrogen.

14. A composition as claimed in claim 13, wherein the radicals R³-R¹² are selected from the group consisting of fluorine, chlorine, bromine, iodine, methyl, ethyl, propyl, butyl, trifluoromethyl, trifluoromethoxy and difluoromethoxy.

15. A composition as claimed in any of claims 1-14 selected from the group of the following compounds:
1-anilino-3-chloro-4-phenylpyrrol-2,5-dione,
1-anilino-3-methylthio-4-phenylpyrrol-2,5-dione,
3-chloro-4-(4-chlorophenyl)-1-(N-methyl-N-phenylamino)-pyrrol - 2,5-dione,
1-anilino-3-methylsulfinyl-4-phenylpyrrol-2,5-dione,
1-anilino-3-methylsulfonyl-4-phenylpyrrol-2,5-dione,
1-anilino-3-bromo-4-phenylpyrrol-2,5-dione.

16. A compound of the formula II 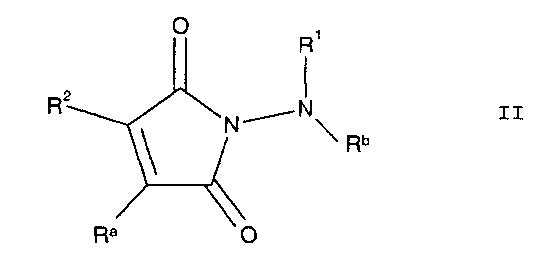 where the radicals R¹, R², R^{a} and R^{b} are as defined below:
R¹ is hydrogen, C₁-C₄-alkyl, formyl;
R² is halogen, C₁-C₆-alkoxy, C₁-C₆-alkylthio, C₁-C₆-alkylsulfonyl, C₁-C₆-alkylsulfinyl or halo-C₁-C₆-alkoxy;
R^{a} is phenyl, which may be mono- or polysubstituted, preferably mono- or disubstituted, by halogen, or by a phenyl group which for its part may also be substituted by halogen or C₁-C₄-alkyl;
R^{b} is phenyl, which may be mono- or polysubstituted, preferably mono- to tetrasubstituted, by halogen, C₁-C₆-alkyl, C₁-C₄-haloalkyl, C₁-C₄-haloalkoxy.

17. The use of fungicidal compositions as claimed in any of claims 1 - 15 or of compounds of the formula II as claimed in claim 16 for controlling harmful fungi in agriculture.

18. A method for controlling harmful fungi, wherein the harmful fungi, their habitat or the plants, areas, materials or spaces to be kept free from them are treated with a fungicidally effective amount of a composition as claimed in any of claims 1 - 15 or of compounds of the formula II as claimed in claim 16.

19. An agrochemical combination preparation, comprising as active compounds compounds of the formula I as claimed in any of claims 1 - 15 or compounds of the formula II as claimed in claim 16 and at least one further fungicidally active compound.

20. The use of fungicidally active compounds in combination with at least one active compound of the formula I as claimed in any of claims 1 - 15 or of compounds of the formula II as claimed in claim 16 for controlling harmful fungi in agriculture.

## Revendications

1. Composition agrochimique possédant une activité fongicide, qui contient, à titre de principes actifs, des composés répondant à la formule (1) 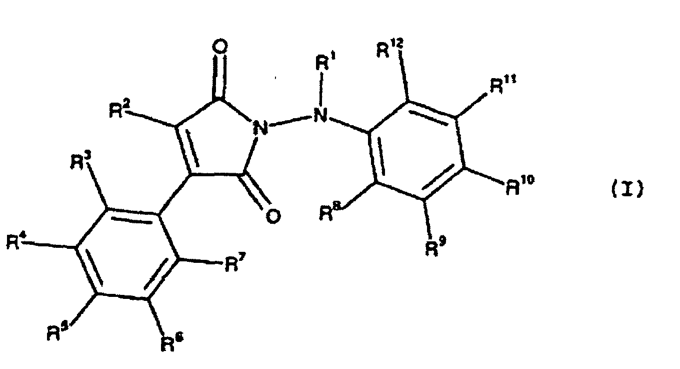 dans laquelle les radicaux possèdent les significations suivantes :
R¹ représente un atome d'hydrogène, un groupe alkyle en C₁-C₆, un groupe alkyl(en C₁-C₆)carbonyle, un groupe formyle ou un groupe halogénoalkyl(en C₁-C₆)carbonyle ;
R² représente un atome d'halogène, un groupe alkyl(en C₁-C₆)thio, un groupe alcoxy en C₁-C₆, un groupe cycloalkyle(en C₃-C₆)alcoxy en C₁-C₆, un groupe alcoxy(en C₁-C₆)alkyle en C₁-C₆, un groupe halogénoalcoxy en C₁-C₆, un groupe alkyl(en C₁-C₆)sulfonyle, un groupe alkyl(en C₁-C₆)sulfinyle, un groupe halogénoalkyl(en C₁-C₆)sulfonyle, un groupe cyano ou un radical NR¹³R¹⁴ ;
R³ - R¹² représentent un atome d'hydrogène, un atome d'halogène, un groupe cycloalkyle en C₁-C₈, un groupe alkyle en C₁-C₆, un groupe halogénoalkyle en C₁-C₆, un groupe alcoxy en C₁-C₆, un groupe halogénoalcoxy en C₁-C₆, un groupe alkyl(en C₁-C₆)sulfonyle, un groupe halogénoalkyl(en C₁-C₆)sulfonyle, un groupe formyle, un groupe alkyl(en C₁-C₆)carbonyle, un groupe cyano, un groupe alkyl(en C₁-C₆)thio ou un groupe phényle qui peut éventuellement être substitué par des atomes d'halogène, par des groupes alkyle en C₁-C₆ ou par des groupes halogénoalkyle en C₁-C₆;
R¹³ représente un atome d'hydrogène, un groupe alkyle en C₁-C₆,
R¹⁴ représente un groupe alkyle en C₁-C₆, un groupe cycloalkyle en C₁-C₈ ou représente, ensemble avec R¹³ et avec l'atome d'azote auquel ils sont liés, un noyau hétérocyclique à cinq ou six membres, saturé ou insaturé, qui contient un ou deux hétéroatomes choisis parmi le groupe comprenant un atome d'azote ou un atome d'oxygène ;
ainsi que leurs sels utilisables en agriculture.

2. Composition selon la revendication 1, dans laquelle R¹ représente un atome d'hydrogène, un groupe alkyle en C₁-C₆, un groupe alkyl(en C₁-C₆)carbonyle ou un groupe formyle.

3. Composition selon la revendication 2, dans laquelle R¹ représente un atome d'hydrogène, un groupe méthyle, un groupe formyle ou un groupe acétyle.

4. Composition selon l'une quelconque des revendications 1 à 3, dans laquelle R² représente un atome d'halogène, un groupe alkyl(en C₁-C₆)thio, un groupe alkyl(en C₁-C₆)sulfonyle, un groupe alkyl(en C₁-C₆)sulfinyle ou un groupe halogénoalcoxy en C₁-C₆.

5. Composition selon la revendication 4, dans laquelle R² représente un atome de chlore, un atome de brome, un groupe méthylthio, un groupe méthylsulfonyle, un groupe méthylsulfinyle ou un groupe difluorométhoxy.

6. Composition selon l'une quelconque des revendications 1 à 5, dans laquelle un ou plusieurs des radicaux R³ - R¹² ont les significations suivantes : un atome de fluor, un atome de chlore, un groupe méthyle, un groupe éthyle, un groupe propyle, un groupe butyle, un groupe trifluorométhyle, un groupe difluorométhyle, un groupe trifluorométhoxy, un groupe difluorométhoxy, un groupe méthoxy, un groupe méthylthio, un groupe cyano et au moins trois radicaux parmi les radicaux R³ - R¹² représentent un atome d'hydrogène.

7. Composition selon la revendication 6, dans laquelle de quatre à neuf radicaux parmi les radicaux R³ - R¹² représentent un atome d'hydrogène.

8. Composition selon l'une quelconque des revendications 1 à 7, dans laquelle de deux à cinq radicaux parmi les radicaux R⁸ - R¹² représentent un atome d'hydrogène.

9. Composition selon la revendication 8, dans laquelle trois ou quatre radicaux parmi les radicaux R⁸ - R¹² représentent un atome d'hydrogène.

10. Composition selon l'une quelconque des revendications 1 à 9, dans laquelle de deux à cinq radicaux parmi les radicaux R³ - R⁷ représentent un atome d'hydrogène.

11. Composition selon la revendication 10, dans laquelle trois ou quatre radicaux parmi les radicaux R³ - R⁷ représentent un atome d'hydrogène.

12. Composition selon l'une quelconque des revendications 1 à 11, dans laquelle au moins deux radicaux parmi les radicaux R⁸ - R¹² et au moins deux radicaux parmi les radicaux R³ - R⁷ représentent un atome d'hydrogène.

13. Composition selon l'une quelconque des revendications 1 à 12, dans laquelle un radical, deux radicaux ou trois radicaux parmi les radicaux R³ - R¹² représentent un atome d'halogène, un groupe alkyle en C₁-C₆, un groupe halogénoalkyle en C₁-C₆, un groupe alcoxy en C₁-C₆ ou un groupe halogénoalcoxy en C₁-C₆, les autres radicaux R³ - R¹² représentant un atome d'hydrogène.

14. Composition selon la revendication 13, dans laquelle les radicaux R³ - R¹² sont choisis parmi le groupe comprenant un atome de fluor, un atome de chlore, un atome de brome, un atome d'iode, un groupe méthyle, un groupe éthyle, un groupe propyle, un groupe butyle, un groupe trifluorométhyle, un groupe trifluorométhoxy ou un groupe difluorométhoxy.

15. Composition selon l'une quelconque des revendications 1 à 14 choisie parmi le groupe des composés ci-après:
la 1-anilino-3-chloro-4-phényl-pyrrole-2,5-dione;
la 1-anilino-3-méthylthio-4-phényl-pyrrole-2,5-dione;
la 3-chloro-4-(4-chlorophényl)-1-(N-méthyl-N-phénylamino)-pyrrole-2,5-dione;
la 1-anilino-3-méthylsulfinyl-4-phényl-pyrrole-2,5-dione;
la 1-anilino-3-méthylsulfonyl-4-phényl-pyrrole-2,5-dione;
la 1-anilino-3-bromo-4-phényl-pyrrole-2,5-dione.

16. Composés répondant à la formule II 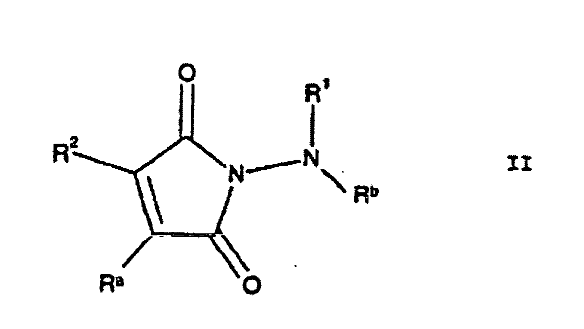 dans laquelle les radicaux R¹, R², R^{a} et R^{b} ont les significations ci-après :
R¹ représente un atome d'hydrogène, un groupe alkyle en C₁-C₄, un groupe formyle ;
R² représente un atome d'halogène, un groupe alcoxy en C₁-C₆, un groupe alkyl(en C₁-C₆)thio, un groupe alkyl(en C₁-C₆)sulfonyle, un groupe alkyl(en C₁-C₆)sulfinyle ou un groupe halogénoalcoxy en C₁-C₆;
R^{a} représente un groupe phényle qui peut être substitué une ou plusieurs fois, de préférence une ou deux fois par un atome d'halogène ou par un groupe phényle qui peut, quant à lui, être également substitué par un atome d'halogène ou par un groupe alkyle en C₁-C₄;
R^{b} représente un groupe phényle qui peut être substitué une ou plusieurs fois, de préférence de une à quatre fois par un atome d'halogène, par un groupe alkyle en C₁-C₆, par un groupe halogénoalkyle en C₁-C₄, par un groupe halogénoalcoxy en C₁-C₄.

17. Utilisation d'agents fongicides selon l'une quelconque des revendications 1 à 15 ou de composés répondant à la formule II selon la revendication 16 pour lutter contre des champignons nuisibles en agriculture.

18. Procédé pour lutter contre des champignons nuisibles, **caractérisé en ce qu'**on traite les champignons nuisibles, leur biotope ou les plantes, les surfaces, les matières ou les pièces qui doivent en être libérés, avec une quantité efficace du point de vue fongicide d'un agent selon l'une quelconque des revendications 1 à 15 ou de composés répondant à la formule II selon la revendication 16.

19. Préparations combinatoires agrochimiques contenant, à titre de principes actifs, des composés répondant à la formule 1 selon les revendications 1 à 15 ou des composés répondant à la formule II selon la revendication 16, et au moins un autre principe actif fongicide.

20. Utilisation de principes actifs fongicides en combinaison avec au moins un principe actif répondant à la formule 1 selon l'une quelconque des revendications 1 à 15 ou de composés répondant à la formule Il selon la revendication 16, pour lutter contre des champignons nuisibles en agriculture.
